# EUROPEAN PATENT APPLICATION

(11) **EP 4 741 499 A1**
(43) Date of publication of application: **13.05.2026**
(21) Application number: 24383218.5
(22) Date of filing: 07.11.2024
(51) Int. Cl.: C12N 15/10, C12N 15/66

(54) **PROTECTED DNA AND METHODS FOR THE PRODUCTION THEREOF**

(71) Applicant: 4basebio UK Ltd, Over, Cambridge CB24 5QE (GB)
(72) Inventor: BOUCHAREB, Amine, Cambridge CB24 5QE (GB); RISTIN, Anca-Paula, Cambridge CB24 5QE (GB); WALKER, Amy, Cambridge CB24 5QE (GB); PICHER, Ángel, 28049 Madrid (ES); LANCKRIET, Heikki, Cambridge CB24 5QE (GB)
(74) Representative: Boult Wade Tennant LLP

(57) **Abstract**

Protected DNA comprising a single-stranded DNA (ssDNA) cassette is provided. Further provided are uses of the protected DNA, methods for producing protected DNA, products generated in performing such methods (including intermediate and final products), and kits for use in such methods.

## Description

### TECHNICAL FIELD

The present invention relates to protected DNA comprising a single-stranded DNA (ssDNA) cassette and uses thereof. The present invention also relates to methods for producing a protected DNA, products generated in performing such methods (including intermediate and final products), and kits for use in such methods.

### BACKGROUND

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

For linear molecules, one of the proposed solutions includes the use of phosphorothioated nucleotides (i.e. 2'-deoxynucleotides-5'-(α-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Phosphorothioate modifications have also been used in the context of a linear double-stranded polynucleotide chain (e.g. double-stranded DNA) to cap the ends of the polynucleotide chain to increase resistance to exonuclease digestion (Putney et al. "A DNA fragment with an alpha-phosphorothioate nucleotide at one end is asymmetrically blocked from digestion by exonuclease III and can be replicated in vivo." Proceedings of the National Academy of Sciences 78.12 (1981): 7350-7354). To cap the ends of the polynucleotide chain, the ends are digested with a restriction enzyme and treated with a DNA polymerase and a mixture of deoxyribonucleotide triphosphates (dNTPs), at least one type of which is a phosphorothioated nucleotide complementary to a nucleotide in the overhanging strand. Since DNA polymerases add nucleotides in the 5' to 3' direction, the result of this treatment is a blunt-ended polynucleotide fragment with a phosphorothioated nucleotide located at the 3'-end of each strand (i.e. in "the cap").

In addition, in therapeutic nucleic acids, phosphorothioated nucleotides are incorporated into short, single-stranded polynucleotide chains. For example, an antisense oligonucleotide fomivirsen is a 21-mer phosphorothioate oligodeoxynucleotide used to treat cytomegalovirus retinitis (Stein and Castanotto, "FDA-approved oligonucleotide therapies in 2017." Molecular Therapy 25.5 (2017): 1069-1075). Similarly, pegaptanib (brand name Macugen) is a short (27-nucleotides) aptamer with a phosphorothioate 3'-3' deoxythymidine cap used for treating age-related macular degeneration of the retina.

However, chemical synthesis of such single-stranded polynucleotides is limited to the generation of relatively short nucleotides. Thus, chemical synthesis of longer single-stranded polynucleotides (>100 mer) presents a number of challenges. For example, as the DNA length starts to exceed 100 nucleotides, the yield of desired products becomes limited by side reactions and even modest inefficiencies within the stepwise chemical reactions have large effects. WO2024/017978 describes methods which are capable of generating long single-stranded DNA molecules particularly longer single-stranded DNA molecules which are stable for *in vivo* applications. In the context of *in vivo* applications, however, the yield and purity of the synthetic product is crucially important. Thus, a need exists for efficient methods which are capable of generating long single-stranded DNA molecules at high yield and purity.

### DESCRIPTION

The invention provides improved methods for producing a protected DNA comprising a single-stranded DNA (ssDNA) cassette (e.g. a long ssDNA cassette). The methods may generate a higher yield of the protected DNA and/or a higher purity of the protected DNA compared to known methods (e.g. the methods described in WO2024/017978). The inventors have surprisingly discovered that the use of T7 DNA ligase in the methods described herein produces a higher yield of the protected DNA. The inventors have also surprisingly discovered that an exonuclease mixture used during the step of digestion as described herein improves the purity of the protected DNA. Accordingly, the methods of the invention, which are based on these surprising discoveries, may generate protected DNA with a higher yield and a higher purity.

The invention provides a method for producing a protected DNA comprising a single-stranded DNA (ssDNA) cassette, wherein the protected DNA comprises a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette. The nuclease-resistant nucleotides are preferably exonuclease-resistant nucleotides e.g. phosphorothioated nucleotides. The location of exonuclease-resistant nucleotides at the 5' and 3' ends of and/or outside of the ssDNA cassette protects the ssDNA cassette from exonuclease digestion. For example, it provides protection from digestion by exonucleases that cleave the 3'-end nucleotides (e.g. exonuclease III) and exonucleases that cleave the 5'-end nucleotides (e.g. exonuclease VIII).

The enhanced resistance to exonuclease digestion extends the life of the ssDNA cassette in a cell (i.e. the ssDNA cassette has enhanced resistance to intracellular exonucleases) and in a cell-free system (i.e. the ssDNA cassette has enhanced resistance to extracellular exonucleases).

As a result of its protection, the ssDNA cassette of the protected DNA may also have prolonged in vivo expression when compared to a linear double-stranded DNA product that does not contain protected nucleotides.

The protected DNA of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear double-stranded DNA product of the present invention particularly suitable for use in a pharmaceutical composition.

In addition, the present inventors have discovered a method for efficient introduction of protected nucleotides at both the 3'-end and the 5'-end of a ssDNA cassette to form a protected DNA. The protected DNA may have improved purity. The invention also provides a method for producing a protected DNA comprising a single-stranded DNA (ssDNA) cassette, wherein the method comprises: (a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette; and (b) digesting the second strand of the partially protected dsDNA with an exonuclease mixture thereby generating the protected DNA. The exonuclease mixture may comprise at least a 5' to 3' exonuclease and a 3' to 5' exonuclease. The exonuclease mixture may comprise at least three exonucleases, wherein at least one of the at least three exonucleases is a 5' to 3' exonuclease and at least one of the at least three exonucleases is a 3' to 5' exonuclease. The exonuclease mixture may comprise at least four exonucleases, wherein at least one of the at least four exonucleases is a 5' to 3' exonuclease and at least one of the at least four exonucleases is a 3' to 5' exonuclease. The second strand may comprise an excisable nucleotide which is recognised by a DNA glycosylase to produce an abasic site which is excised by an enzyme having AP endonuclease activity, or excised directly by a DNA glycosylase. The second strand may comprise an abasic site. The second strand may comprise a target sequence for a nicking endonuclease.

The partially protected dsDNA may be generated by digesting a precursor dsDNA with an endonuclease and ligating first and second adaptor molecules to the digested precursor dsDNA. Preferably, ligation is performed using T7 DNA ligase. The first and second adaptor molecules may comprise exonuclease-resistant nucleotides e.g. phosphorothioated nucleotides. The first and/or second adaptor molecules may comprise excisable nucleotides.

Thus, the invention provides a method for producing a protected DNA comprising a single-stranded DNA (ssDNA) cassette, wherein the method comprises:
(a) generating a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1, wherein the step of generating the partially protected dsDNA comprises:
   (i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
   (ii) digesting the precursor dsDNA with the endonuclease to generate a digested precursor dsDNA;
   (iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1, and wherein the ligase is T7 DNA ligase; and
   (iv) ligating the first adaptor molecule to a first end of the digested precursor dsDNA and ligating the second adaptor molecule to a second end of the digested precursor dsDNA thereby generating the partially protected dsDNA; and
(b) digesting the second strand of the partially protected dsDNA with an exonuclease mixture thereby generating the protected DNA, wherein the exonuclease mixture comprises at least a 5' to 3' exonuclease and a 3' to 5' exonuclease.

The invention also provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease mixture thereby generating the protected DNA, wherein the exonuclease mixture comprises at least three exonucleases, wherein at least one of the at least three exonucleases is a 5' to 3' exonuclease and at least one of the at least three exonucleases is a 3' to 5' exonuclease.

Importantly, the methods enable the high fidelity and/or high yield production of long and stable ssDNA cassettes that have a wide range of applications and uses, as provided herein. The methods also enable the production of products (i.e. protected DNA) of high purity. The protected DNA obtainable by the methods of the invention has broad industrial application, as described in WO2024/017978 (which is incorporated herein by reference). For example, the protected DNA can be used in (1) *in vitro* transcription, (2) production of a protein in a cell or a cell-free system, (3) production of a viral or non-viral delivery system, (4) gene therapy, (5) cell therapy, (6) production of a vaccine, (7) gene editing, and/or (8) production of a CAR-T cell.

### 1. Protected DNA

The methods of the invention produce a protected DNA comprising a single-stranded DNA (ssDNA) cassette, wherein the protected DNA comprises a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette.

The protected DNA has enhanced resistance to exonuclease digestion. The enhanced resistance to exonuclease digestion may extend the life of the ssDNA cassette in a cell (i.e. the ssDNA cassette may have enhanced resistance to intracellular exonucleases) and/or the enhanced resistance to exonuclease digestion may extend the life of the ssDNA cassette in a cell-free system (i.e. the ssDNA cassette may have enhanced resistance to extracellular exonucleases). The ssDNA cassette is protected from digestion by exonucleases that cleave the 3'-end nucleotides (e.g. exonuclease I), exonucleases that cleave the 5'-end nucleotides (e.g. exonuclease VIII) and exonucleases that cleave both the 3' and 5' end nucleotides (e.g. exonuclease VII). Thus, the ssDNA cassette may have prolonged *in vivo* expression when compared to DNA that does not comprise nuclease-resistant nucleotides as described herein.

The protected DNA may have additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes protected DNA of the present invention particularly suitable for use in a pharmaceutical composition.

The protected DNA comprising a single-stranded DNA (ssDNA) cassette may comprise a nuclease-resistant nucleotide at the 5' end of the ssDNA cassette or 5' of the ssDNA cassette, and a nuclease-resistant nucleotides at the 3'-end of the ssDNA cassette or 3' of the ssDNA cassette, wherein the cassette comprises at least 100 nucleotides. The protected DNA comprising a single-stranded DNA (ssDNA) cassette may comprise at least 3 nuclease-resistant nucleotides at the 5' end of the ssDNA cassette or 5' of the ssDNA cassette, and at least 3 nuclease-resistant nucleotides at the 3'-end of the ssDNA cassette or 3' of the ssDNA cassette, optionally wherein the ssDNA cassette comprises at least 100 nucleotides. The protected DNA comprising a single-stranded DNA (ssDNA) cassette may comprise at least 5 nuclease-resistant nucleotides at the 5' end of the ssDNA cassette or 5' of the ssDNA cassette, and at least 5 nuclease-resistant nucleotides at the 3'-end of the ssDNA cassette or 3' of the ssDNA cassette, optionally wherein the ssDNA cassette comprises at least 100 nucleotides. The protected DNA comprising a single-stranded DNA (ssDNA) cassette may comprise x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1, optionally wherein the ssDNA cassette comprises at least 100 nucleotides.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

"Protected DNA" as used herein refers to the protected DNA comprising a single-stranded DNA (ssDNA) cassette unless specified otherwise.

The protected DNA may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease T and/or exonuclease VII) and/or 5'-end exonuclease digestion (e.g. by exonuclease VIII, exonuclease VII, RecJf, RecJ, T7 exonuclease, Lambda exonuclease and/or T5 exonuclease).

The protected DNA comprises nuclease-resistant nucleotides (i.e. protected nucleotides), such as phosphorothioated nucleotides.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to exonuclease digestion. For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The protected DNA may comprise a double stranded region 5' of the cassette. The protected DNA may comprise a double-stranded region 3' of the cassette. The protected DNA may comprise a double-stranded region 5' of the cassette and a double-stranded region 3' of the cassette. Protected DNA comprising double-stranded regions may provide higher efficiency when used as a repair template (or editing template) for CRISPR-Cas mediated homology directed repair (HDR) than a single-stranded repair template. Such a partially double-stranded DNA molecule may also provide lower toxicity compared to a (fully) double-stranded DNA molecule.

The protected DNA may be partially double-stranded. The protected DNA may comprise a portion that is double-stranded and a portion that is single-stranded (i.e. at least the ssDNA cassette).

The protected DNA may be a single-stranded DNA molecule i.e. the protected DNA may not comprise any double-stranded regions.

The protected DNA may comprise a spacer for example in the cassette and/or between the cassette and the nuclease-resistant nucleotides 3' and 5' of the cassette. The spacer may be at least 10, at least 20, at least 30, at least 40, at least 50, at least 60, at least 70, at least 80, at least 90, at least 100, at least 125, at least 150, at least 175, or at least 200 nucleotides long. The spacer may improve cell transfection yields.

The protected DNA may comprise at least 50, at least 100, at least 150, at least 200, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the protected DNA comprises at least 200 nucleotides.

The ssDNA cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the ssDNA cassette comprises at least 100 nucleotides.

The ssDNA cassette may comprise at least 100 nucleotides, at least 200 nucleotides, 500 nucleotides, 1000 nucleotides, 5000 nucleotides or 10000 nucleotides.

The ssDNA cassette may be a single cassette. The term "single cassette" as used herein is intended to encompass a product that does not comprise or consist of a plurality of cassettes. That is to say that the protected DNA comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The ssDNA cassette may comprise a coding sequence. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000, at least 15,000, or at least 20,000 nucleotides. Preferably, the gene comprises at least 200 nucleotides.

The ssDNA cassette may comprise at least a portion of a promoter and a coding sequence. The cassette may comprise a promoter and a coding sequence. The ssDNA cassette may comprise a promoter, a coding sequence, a ribosomal binding site and a translational termination sequence. The ssDNA cassette may additionally comprise sequences aiding protein expression, such as a cap-independent translation element. The ssDNA cassette may comprise (or encode) a repair template (or editing template). The repair template (or editing template) may be for use in CRISPR-Cas mediated homology directed repair (HDR). The ssDNA cassette may encode CRISPR guide RNA. The ssDNA cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The protected DNA may not comprise a hairpin, a loop or a stem-loop structure.

The protected DNA may additionally comprise a plurality of protected nucleotides (e.g. phosphorothioated nucleotides) at internal positions. For example, the protected DNA may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions. Preferably, the protected DNA comprises at least 2 protected nucleotides (e.g. phosphorothioated nucleotides) at internal positions.

The internal positions may not be located between the second and penultimate nucleotide of the protected DNA.

The invention provides a protected DNA obtainable by any of the methods described herein.

Aspects of the invention described above in relation to the protected DNA apply *mutatis mutandis* to all aspects of the invention described herein.

### 2. Partially protected double-stranded DNA

The partially protected double-stranded DNA (dsDNA) may comprise a first strand and a second strand, wherein the first strand comprises: (i) a cassette; (ii) a nuclease-resistant nucleotide at the 5' end of the cassette or 5' of the cassette; and (iii) a nuclease-resistant nucleotide at the 3'-end of the cassette or 3' of the cassette.

The partially protected double-stranded (dsDNA) may be used to produce the protected DNA.

The partially protected double-stranded DNA (dsDNA) may comprise a first strand and a second strand, wherein the first strand comprises: (i) a cassette; (ii) a nuclease-resistant nucleotide at the 5' end of the cassette or 5' of the cassette; and (iii) a nuclease-resistant nucleotides at the 3'-end of the cassette or 3' of the cassette; and wherein the second strand of the dsDNA molecule does not comprise any nuclease-resistant nucleotides. The second strand may comprise an excisable nucleotide. As used herein, "excisable nucleotide" means a nucleotide that is recognised by an enzyme (e.g. a DNA glycosylase) involved in DNA repair as damaged and is modified and excised from the DNA molecule. The excisable nucleotide may be an oxidised base, an alkylated base, a deaminated base, or uracil. The second strand may comprise an excisable nucleotide and the second strand may also comprise protected nucleotides.

The second strand may comprise an AP site (or an abasic site; used herein interchangeably). An AP site is recognised and an incision made in the DNA molecule at that site, leaving it open for exonuclease digestion.

The partially protected double-stranded DNA (dsDNA) may comprise a first strand and a second strand, wherein the first strand comprises: (i) a cassette; (ii) a nuclease-resistant nucleotide at the 5' end of the cassette or 5' of the cassette; and (iii) a nuclease-resistant nucleotides at the 3'-end of the cassette or 3' of the cassette; and wherein the second strand of the dsDNA molecule may comprise nuclease-resistant nucleotides. The second strand may comprise an excisable nucleotide or an abasic site. As used herein, "excisable nucleotide" means a nucleotide that is recognised by an enzyme (e.g. a DNA glycosylase and/or an AP endonuclease) involved in DNA repair as damaged and is modified and excised from the DNA molecule. The excisable nucleotide may be an oxidised base, an alkylated base, a deaminated base, or uracil.

The second strand may comprise a target sequence for a nicking endonuclease. The second strand may comprise a target sequence for a nicking endonuclease and the second strand may not comprise protected nucleotides. The second strand may comprise a target sequence for a nicking endonuclease and the second strand may also comprise protected nucleotides.

The partially protected dsDNA may comprise a first strand and a second strand, wherein the first strand comprises: (i) a cassette, (ii) at least 3 nuclease-resistant nucleotides at the 5' end of the cassette or 5' of the cassette; and (iii) at least 3 nuclease-resistant nucleotides at the 3'-end of the cassette or 3' of the cassette. The second strand of the dsDNA molecule may not comprise any nuclease-resistant nucleotides. The second strand may comprise at least 2 excisable nucleotides. The at least 2 excisable nucleotides are preferably the same. The second strand may comprise at least 2 excisable nucleotides and the second strand may also comprise protected nucleotides. The partially protected dsDNA may comprise a first strand and a second strand, wherein the first strand comprises: (i) a cassette, (ii) at least 5 nuclease-resistant nucleotides at the 5' end of the cassette or 5' of the cassette; and (iii) at least 5 nuclease-resistant nucleotides at the 3'-end of the cassette or 3' of the cassette. The second strand of the dsDNA molecule may not comprise any nuclease-resistant nucleotides. The second strand may comprise at least 2 excisable nucleotides. The at least 2 excisable nucleotides are preferably the same. The second strand may comprise at least 2 excisable nucleotides and the second strand may also comprise protected nucleotides.

The partially protected dsDNA may comprise a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1. The second strand of the dsDNA molecule may not comprise any nuclease-resistant nucleotides. The second strand may comprise n excisable nucleotides. The n excisable nucleotides are preferably the same. The second strand may comprise n excisable nucleotides and the second strand may also comprise protected nucleotides. The second strand may comprise 3 or 5 protected nucleotides at the 3' end or 3' of the cassette. The second strand may comprise 3 or 5 protected nucleotides at the 5' end or 5' of the cassette. The partially protected dsDNA may comprise a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1. The second strand of the dsDNA molecule may not comprise any nuclease-resistant nucleotides. The second strand may comprise n abasic sites. The n abasic sites are preferably the same. The second strand may comprise n abasic sites and the second strand may also comprise protected nucleotides. The second strand may comprise 3 or 5 protected nucleotides at the 3' end or 3' of the cassette. The second strand may comprise 3 or 5 protected nucleotides at the 5' end or 5' of the cassette.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

The second strand of the partially protected dsDNA is hybridised to the first strand of the partially protected dsDNA. The second strand of the partially protected dsDNA may comprise a sequence complementary to the cassette of the first strand of the partially protected dsDNA. The second strand of the partially protected dsDNA may comprise a sequence complementary to the cassette, y' nuclease-resistant nucleotides at the 5' end of the sequence complementary to the cassette or 5' of the sequence complementary to the cassette, and x' nuclease-resistant nucleotides at the 3'-end of the sequence complementary to the cassette or 3' of the sequence complementary to the cassette.

x' and y' may be independently selected from 0, at least 1, at least 2, at least 3, at least 4, at least 5. x' and y' may be the same or different. x' may be 0 and y' may be 3 or *vice versa.* x' may be 0 and y' may be 5 or *vice versa.*

The second strand may comprise n excisable nucleotides. The n excisable nucleotides are preferably the same. The second strand may comprise n excisable nucleotides and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or or 3' of the cassette. The n excisable nucleotides are preferably at the same end of the cassette as the protected nucleotides, i.e. the excisable nucleotides and the protected nucleotides are 5' of or at the 5' end of the cassette, or the excisable nucleotides and the protected nucleotides are 3' of or at the 3' end of the cassette. There may be at least 1, at least 2, at least 3, at least 4, at least 5, at least 6, at least 7 or at least 8 nucleotides between the protected nucleotides and the excisable nucleotides. The protected nucleotides are preferably to the 3' of the excisable nucleotides when the protected nucleotides and the excisable nucleotides are at the 3' end of or 3' of the cassette. The protected nucleotides are preferably to the 5' of the excisable nucleotides when the protected nucleotides and the excisable nucleotides are at the 5' end of or 5' of the cassette.

The second strand may comprise n abasic sites. The n abasic sites are preferably the same. The second strand may comprise n abasic sites and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or 3' of the cassette.

The n abasic sites are preferably at the same end of the cassette as the protected nucleotides, i.e. the abasic sites and the protected nucleotides are 5' of or at the 5' end of the cassette, or the abasic sites and the protected nucleotides are 3' of or at the 3' end of the cassette. There may be at least 1, at least 2, at least 3, at least 4, at least 5, at least 6, at least 7 or at least 8 nucleotides between the protected nucleotides and the abasic sites. The protected nucleotides are preferably to the 3' of the abasic sites when the protected nucleotides and the excisable nucleotides are at the 3' end of or 3' of the cassette. The protected nucleotides are preferably to the 5' of the abasic sites when the protected nucleotides and the excisable nucleotides are at the 5' end of or 5' of the cassette.

The second strand may comprise at least 3 protected nucleotides and at least 1 excisable nucleotides at the 3' end of the cassette or 3' of the cassette. The second strand may comprise at least 4 protected nucleotides and at least 1 excisable nucleotides at the 3' end of the cassette or 3' of the cassette. The second strand may comprise at least 5 protected nucleotides and at least 1 excisable nucleotides at the 3' end of the cassette or 3' of the cassette. The second strand may comprise at least 3 protected nucleotides and at least 2 excisable nucleotides at the 3' end of the cassette or 3' of the cassette. The second strand may comprise at least 4 protected nucleotides and at least 2 excisable nucleotides at the 3' end of the cassette or 3' of the cassette. The second strand may comprise at least 5 protected nucleotides and at least 2 excisable nucleotides at the 3' end of the cassette or 3' of the cassette. The second strand may comprise at least 3 protected nucleotides and at least 1 excisable nucleotides at the 5' end of the cassette or 5' of the cassette. The second strand may comprise at least 4 protected nucleotides and at least 1 excisable nucleotides at the 5' end of the cassette or 5' of the cassette. The second strand may comprise at least 5 protected nucleotides and at least 1 excisable nucleotides at the 5' end of the cassette or 5' of the cassette. The second strand may comprise at least 3 protected nucleotides and at least 2 excisable nucleotides at the 3' end of the cassette or 5' of the cassette. The second strand may comprise at least 4 protected nucleotides and at least 2 excisable nucleotides at the 5' end of the cassette or 5' of the cassette. The second strand may comprise at least 5 protected nucleotides and at least 2 excisable nucleotides at the 5' end of the cassette or 5' of the cassette.

There may be at least 4 nucleotides between the protected nucleotides or abasic sites and the excisable nucleotides or abasic sites. There may be at least 5 nucleotides between the protected nucleotides and the excisable nucleotides or abasic sites. There may be at least 6 nucleotides between the protected nucleotides and the excisable nucleotides or abasic sites.

In the methods described herein, y may be greater than x. For example, y may be at least 3 or at least 5 and x may be at least 1. In the methods described herein, x' may be greater than y'. For example, y' may be 0 and x' may be at least 3, or at least 5. In the methods described herein, x may be greater than y. For example, x may be at least 3 or at least 5 and y may be at least 1. In the methods described herein, y' may be greater than x'. For example, x' may be 0 and y' may be at least 3, or at least 5.

The excisable nucleotide may be 8-oxoguanadine, 2,6-diamino-4-hydroxy-5-formamidopyrimidine (FapyG or FapyA), 3-methyladenine, 7-methylguanosine, hypoxanthine, xanthine or thymidine, uracil, thymine glycol, 5-hydroxycytosine (5-hC), 5-hydroxyuracil (5-hU), 3-methylguanine (3-meG), 3,N4-ethenocytosine or an ethylated base. The second strand may comprise an abasic site, also known as an AP site, which is an apurinic or apyrimidinic site in the DNA that does not have a purine or a pyrimidine.

The partially protected dsDNA may comprise a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1. The second strand of the dsDNA molecule may not comprise any nuclease-resistant (i.e. protected) nucleotides. The second strand may comprise a nicking endonuclease target sequence. The second strand may comprise a nicking endonuclease target sequence and the second strand may also comprise nuclease-resistant nucleotides.

The nicking endonuclease target sequence may be the target sequence of the nicking endonuclease Nt.BspQI, Nt.CviPII, Nt.BstNBI, Nb.BsrDI, Nb.Btsl, Nt.Alwl, Nb.BbvCl, Nt.BbvCl, Nb.Bsml, Nb.BssSI and/or Nt.BsmAI.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

The second strand of the partially protected dsDNA may not comprise any nuclease-resistant nucleotides. The second strand of the partially protected dsDNA may not be resistant to exonuclease digestion. The second strand of the partially protected dsDNA may be susceptible to exonuclease digestion. The second strand may comprise nuclease resistant nucleotides at the 3' end of or 3' of the cassette. The second strand may comprise nuclease resistant nucleotides at the 5' end of or 5' of the cassette.

The second strand may comprise nuclease resistant nucleotides at the 3' end of or 3' of the cassette and the second strand may comprise nuclease resistant nucleotides at the 5' end of or 5' of the cassette. The sequence complementary to the cassette may not comprise any nuclease-resistant nucleotides. The sequence complementary to the cassette may not be resistant to exonuclease digestion. The sequence complementary to the cassette may be susceptible to exonuclease digestion.

The first strand of the partially protected dsDNA may be a sense or a coding strand. The first strand of the partially protected dsDNA may be an antisense or non-coding strand. The second strand of the partially protected dsDNA may be a sense or a coding strand. The second strand of the partially protected dsDNA may be an antisense or non-coding strand.

The second strand of the partially protected dsDNA may not be covalently linked to the first strand of the partially protected dsDNA molecule.

The partially protected dsDNA may comprise a spacer for example in the cassette and/or between the cassette and the nuclease-resistant nucleotides 3' and 5' of the cassette. The spacer may be at least 10, at least 20, at least 30, at least 40, at least 50, at least 60, at least 70, at least 80, at least 90, at least 100, at least 125, at least 150, at least 175, or at least 200 nucleotides long.

The partially protected dsDNA may comprise at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs. Preferably, the partially protected dsDNA comprises at least 200 base pairs.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may be a single cassette. The term "single cassette" as used herein is intended to encompass a product that does not comprise or consist of a plurality of cassettes. That is to say that the partially protected dsDNA comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The partially protected dsDNA may additionally comprise a plurality of protected nucleotides (e.g. phosphorothioated nucleotides) at internal positions. For example, the partially protected DNA may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions. Preferably, the partially protected DNA comprises at least 2 protected nucleotides (e.g. phosphorothioated nucleotides) at internal positions.

The internal positions may not be located between the second and penultimate nucleotide of the partially protected DNA.

The invention provides a partially protected dsDNA obtainable by any of the methods described herein.

Aspects of the invention described above in relation to the partially protected dsDNA apply *mutatis mutandis* to all aspects of the invention described herein.

### 3. Methods for producing protected DNA and partially protected DNA

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette; and
(b) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotides at the 5' end of the cassette or 5' of the cassette, and at least 3 nuclease-resistant nucleotides at the 3'-end of the cassette or 3' of the cassette; and
(b) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotides at the 5' end of the cassette or 5' of the cassette, and at least 5 nuclease-resistant nucleotides at the 3'-end of the cassette or 3' of the cassette; and
(b) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease mixture thereby generating the protected DNA.
The exonuclease mixture may comprise at least a 5' to 3' exonuclease and a 3' to 5' exonuclease. The exonuclease mixture may comprise at least three exonucleases, wherein at least one of the at least three exonucleases is a 5' to 3' exonuclease and at least one of the at least three exonucleases is a 3' to 5' exonuclease. The exonuclease mixture may comprise at least three exonucleases, wherein at least two of the at least three exonucleases are 5' to 3' exonucleases and at least one of the at least three exonucleases is a 3' to 5' exonuclease. The exonuclease mixture may comprise at least three exonucleases, wherein at least one of the at least three exonucleases is a 5' to 3' exonuclease and at least two of the at least three exonucleases are 3' to 5' exonucleases. The exonuclease mixture may comprise at least four exonucleases, wherein at least one of the at least four exonucleases is a 5' to 3' exonuclease and at least one of the at least four exonucleases is a 3' to 5' exonuclease. The exonuclease mixture may comprise at least four exonucleases, wherein at least two of the at least four exonucleases are 5' to 3' exonucleases and at least one of the at least four exonucleases is a 3' to 5' exonuclease. The exonuclease mixture may comprise at least four exonucleases, wherein at least one of the at least four exonucleases is a 5' to 3' exonuclease and at least two of the at least four exonucleases are 3' to 5' exonucleases. Preferably, the exonuclease mixture comprises at least four exonucleases, wherein at least two of the at least four exonucleases are 5' to 3' exonucleases and at least two of the at least four exonucleases are 3' to 5' exonucleases.

The 5' to 3' exonuclease(s) may be Lambda exonuclease and/or T7 exonuclease. The 3' to 5' exonuclease(s) may be exonuclease I and/or exonuclease III. The 5' to 3' exonuclease(s) may be exonuclease VIII.

The exonuclease mixture may comprise at least two exonucleases selected from a group of exonuclease I, exonuclease III, T7 exonuclease, and Lambda exonuclease. The exonuclease mixture may comprise at least three exonucleases selected from a group of exonuclease I, exonuclease III, T7 exonuclease, and Lambda exonuclease. The exonuclease mixture may comprise exonuclease I, exonuclease III, T7 exonuclease, and Lambda exonuclease.

The exonuclease mixture may comprise one exonuclease selected from Lambda exonuclease and/or T7 exonuclease, and one exonuclease selected from exonuclease I and/or exonuclease III. For example, the exonuclease mixture may comprise Lambda exonuclease and exonuclease I. For example, the exonuclease mixture may comprise Lambda exonuclease and exonuclease III. For example, the exonuclease mixture may comprise T7 exonuclease and exonuclease I. For example, the exonuclease mixture may comprise T7 exonuclease and exonuclease III.

The exonuclease mixture may comprise Lambda exonuclease, T7 exonuclease and exonuclease I. The exonuclease mixture may comprise Lambda exonuclease, T7 exonuclease and exonuclease III. The exonuclease mixture may comprise Lambda exonuclease, exonuclease I and exonuclease III. The exonuclease mixture may comprise T7 exonuclease, exonuclease I and exonuclease III.

The exonuclease mixture may further comprise exonucleases which are able to cleave DNA in both 3' to 5' and 5' to 3' directions. For example, the exonuclease mixture may comprise exonuclease V.

The exonucleases from the exonuclease mixture may be added sequentially or simultaneously. Preferably the exonucleases from the exonuclease mixture are added simultaneously.

x and y may be independently selected from at least 1, at least 2, at least 3, at least 4 and at least 5. x and y may be different or the same. x and y may both be at least 3. x and y may both be at least 5. x may be 3 and y be 1 and *vice versa.* x may be 3 and y be 5 and *vice versa.*

The method may enable the efficient production of a large quantity of protected DNA with high purity. The protected DNA produced by the methods described herein may be at least 5%, at least 10%, at least 15%, at least 20%, at least 25%, at least 30%, at least 35%, at least 40%, at least 45%, at least 50%, at least 55%, at least 60%, at least 65%, at least 70%, at least 85%, at least 90%, at least 95%, or at least 100%, purer than the protected DNA produced using a method that uses T4 DNA ligase and/or a single exonuclease (or two exonucleases) e.g. a method described in WO2024/017978. The methods described herein may produce a yield of the protected DNA that is at least 5%, at least 10%, at least 15%, at least 20%, at least 25%, at least 30%, at least 35%, at least 40%, at least 45%, at least 50%, at least 55%, at least 60%, at least 65%, at least 70%, at least 85%, at least 90%, at least 95%, or at least 100%, higher than a method that uses T4 DNA ligase and/or a single exonuclease (or two exonucleases) e.g. a method described in WO2024/017978.

The method may be a cell-free method. The method may enable the production of a product comprising the protected DNA that is substantively free of bacterial contaminants (e.g. remaining after cell lysis).

The partially protected double-stranded DNA (dsDNA) may be any partially protected dsDNA defined herein.

Digesting the second strand of the partially protected dsDNA with an exonuclease mixture may comprise contacting the partially protected dsDNA with an exonuclease mixture to digest the second strand.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette;
(b) nicking the second strand of partially protected dsDNA; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotides at the 5' end of the cassette or 5' of the cassette, and at least 3 nuclease-resistant nucleotides at the 3'-end of the cassette or 3' of the cassette;
(b) nicking the second strand of partially protected dsDNA; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotides at the 5' end of the cassette or 5' of the cassette, and at least 5 nuclease-resistant nucleotides at the 3'-end of the cassette or 3' of the cassette;
(b) nicking the second strand of partially protected dsDNA; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1;
(b) nicking the second strand of partially protected dsDNA; and
(c) digesting the second strand of the partially protected dsDNA with an exonuclease mixture thereby generating the protected DNA.

x and y may be independently selected from at least 1, at least 2, at least 3, at least 4 and at least 5. x and y may be different or the same. x and y may both be at least 3. x and y may both be at least 5. x may be 3 and y be 1 and *vice versa.* x may be 3 and y be 5 and *vice versa.*

For partially protected dsDNA with a second strand comprising an excisable nucleotide, prior to digestion with an exonuclease mixture, the partially protected dsDNA may be contacted with a DNA glycosylase, such as OGG1, MPG, SMUG1,UNG1, MBD4, TDG, MYH1, NTHL1, NEIL1, NEIL2 or NEIL3. Contacting with the DNA glycosylase may be carried out at the same time as digesting with an exonuclease mixture.

For partially protected dsDNA with a second strand comprising a nicking endonuclease target sequence, prior to digesting with an exonuclease mixture, the partially protected dsDNA may be contacted with a nicking endonuclease. The nicking endonuclease may be Nt.BspQI, Nt.CviPII, Nt.BstNBI, Nb.BsrDI, Nb.Btsl, Nt.Alwl, Nb.BbvCI, Nt.BbvCI, Nb.Bsml, Nb.BssSI and/or Nt.BsmAI. Contacting with the nicking endonuclease may be carried out at the same as digesting with the exonuclease mixture.

For partially protected dsDNA with a second strand comprising an abasic site, prior to or at the same time as digesting with an exonuclease mixture, the partially protected dsDNA may be contacted with an AP endonuclease. Alternatively, the exonuclease mixture may comprise an exonuclease with AP endonuclease activity.

Alternatively, digesting the second strand of the partially protected dsDNA with an exonuclease mixture may comprise (a) denaturing the partially protected dsDNA to separate the first and second strands and (b) contacting the second strand with the exonuclease mixture. The partially protected dsDNA may be denatured by means known in the art such as heat and/or alkali conditions.

Heat may include heating the partially protected dsDNA to a temperature of at least 40°C, at least 50°C, at least 60°C, at least 70°C, at least 80°C, or at least 90°C. Preferably, the partially protected dsDNA is heated to at least 95°C. The heating may be carried out for at least 1, at least 3, at least 5, at least 10, at least 15, at least 30 or at least 60 minutes. Preferably, the heating is carried out for at least 3 minutes. Thus, the heating may be at 95°C for at least 3 minutes.

The denatured partially protected dsDNA may be cooled (e.g. to below 20°C, below 10°C or below 5°C) to prevent annealing of the first and second strands. Preferably, the denatured partially protected dsDNA is cooled to around 4°C.

Alkali conditions may be achieved by adding an alkali reagent (e.g. KOH) that creates a pH above 7, 8, 9, 10, 11, 12, 13 or 14. Preferably, an alkali reagent is added that creates a pH above 10. After denaturation, the pH may be lowered by adding an acidic reagent (e.g. HCl). The pH may be lowered by an amount which provides conditions for the exonuclease mixture to be effective at digesting the second strand.

If the partially protected dsDNA is denatured, the exonuclease mixture may comprise an exonuclease which acts on single-stranded DNA (e.g. *E.* coliexonuclease I, exonuclease VII, exonuclease T, RecJf, RecJ).

The step of exonuclease digestion may allow for the removal of any unprotected strands, strands lacking adaptors at both ends and/or remaining adaptor molecules.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises a nuclease resistant nucleotide and the second adaptor molecule comprises a nuclease-resistant nucleotide; and
(d) ligating the first adaptor molecule to a first end of the digested precursor dsDNA and ligating the second adaptor molecule to a second end of the digested precursor dsDNA thereby generating the partially protected dsDNA.

Preferably, the ligase is T7 DNA ligase.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 3 nuclease resistant nucleotides and the second adaptor molecule comprises at least 3 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 5 nuclease resistant nucleotides and the second adaptor molecule comprises at least 5 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1;
(d) nicking the second strand of the protected dsDNA; and
(e) ligating the first adaptor molecule to a first end of the digested precursor dsDNA and ligating the second adaptor molecule to a second end of the digested precursor dsDNA thereby generating the partially protected dsDNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise a nuclease-resistant nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 3 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected DNA.

Preferably, the ligase is T7 DNA ligase.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 5 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease mixture and optionally a DNA glycosylase or a nicking endonuclease;
(f) nicking the second strand of the partially protected dsDNA; and
(g) digesting the second strand of the partially protected dsDNA with the exonuclease mixture thereby generating the protected DNA.

Preferably, the ligase is T7 DNA ligase.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

The second strand of the partially protected dsDNA in the methods described herein may also comprise n excisable nucleotides. n may be at least 1, at least 2, at least 3, at least 4 or at least 5. The methods may therefore further comprise incubating the partially protected DNA with a DNA glycosylase prior to or at the same time as contacting the partially protected dsDNA with an exonuclease mixture.

The second strand of the partially protected dsDNA may comprise n abasic sites, n may be at least 1, at least 2, at least 3, at least 4 or at least 5. The methods may therefore further comprise incubating the partially protected DNA with an AP endonuclease prior to or at the same time as contacting the partially protected dsDNA with an exonuclease mixture. An exonuclease having AP endonuclease activity may perform both of these steps.

The second strand of the partially protected dsDNA in the methods described herein may also comprise a target sequence for a nicking endonuclease. The methods may therefore further comprise incubating the partially protected DNA with a nicking endonuclease prior to or at the same time as contacting the partially protected dsDNA with an exonuclease mixture.

Steps (a) (i.e. contacting a precursor dsDNA comprising a first and a second strand with an endonuclease) to (d) (i.e. ligating the first and second adaptor molecules to the digested precursor dsDNA) may be performed in a single contiguous aqueous volume.

The precursor dsDNA may comprise one or more cleavable (e.g. endonuclease) target sequences. The precursor dsDNA may comprise two cleavable (e.g. endonuclease) target sequences. The one or more endonuclease target sequences may be Type IIS endonuclease target sequences. The one or more endonuclease target sequences may be Bbsl, Bsal, BsmBI, BspQI, BtgZI, Esp3!,Sap!, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV11, BstV2!, Bsul, BtgZI, BtsCI, Btsl-v2, BtsMutl, Bve!, Csel, CspCI, Eam1104I, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, Mlyl, Mmel, Mnll, Mva12691, NmeAIII, PaqCI, PciSI, Pctl, Plel, Ppsl, Psrl, Schl, SfaNI, Taqll, TspDTI and/or TspGWI target sequences.

The method for producing a protected DNA comprising a single-stranded DNA (ssDNA) cassette may comprise:
(a) contacting a precursor dsDNA comprising a first and a second strand with a Type IIS endonuclease, wherein the precursor dsDNA comprises on the first strand a cassette, a Type IIS endonuclease target sequence 5' of the cassette and a Type IIS endonuclease target sequence 3' of the cassette;
(b) digesting the precursor dsDNA with the Type IIS endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected DNA; optionally wherein steps (a) (i.e. contacting a precursor dsDNA comprising a first and a second strand with a Type IIS endonuclease) to (d) (i.e. ligating the first and second adaptor molecules to the digested precursor dsDNA) are performed in a single contiguous aqueous volume.

The precursor dsDNA may be a product of amplification. Preferably, the amplification is rolling circle amplification.

The method may further comprise, before step (a) (i.e. the step of contacting a precursor dsDNA with the endonuclease), a step of amplifying a DNA template to produce the precursor DNA, wherein the DNA template comprises the cassette and the endonuclease target sequences, optionally wherein the DNA template is amplified by rolling circle amplification.

Thus, the method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise a nuclease-resistant nucleotide;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(f) contacting the partially protected dsDNA with an exonuclease mixture; and
(g) digesting the second strand of the partially protected dsDNA with the exonuclease mixture thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 3 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(f) contacting the partially protected dsDNA with an exonuclease mixture; and
(g) digesting the second strand of the partially protected dsDNA with the exonuclease mixture thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 5 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(f) contacting the partially protected dsDNA with an exonuclease mixture; and
(g) digesting the second strand of the partially protected dsDNA with the exonuclease mixture thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease mixture; and
(g) digesting the second strand of the partially protected dsDNA with the exonuclease mixture thereby generating the protected DNA.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

Steps (b) (i.e. contacting the precursor dsDNA comprising a first and a second strand with an endonuclease) to (e) (i.e. ligating the first and second adaptor molecules to the digested precursor dsDNA) may be performed in a single contiguous aqueous volume.

The methods described above may also include a step of nicking the second strand of the partially protected dsDNA prior to digestion with an exonuclease mixture. The nicking may be performed at the same time as contacting the second strand with the exonuclease mixture.

If the second strand of the partially protected dsDNA comprises excisable nucleotides the methods may further comprise a step of nicking the second strand by contacting the partially protected dsDNA with a DNA glycosylase prior to or at the same time as contacting the partially protected dsDNA with an exonuclease mixture.

If the second strand of the partially protected dsDNA comprises abasic sites, the methods may further comprise the step of nicking the second strand by contacting the partially protected DNA with an AP endonuclease prior to or at the same time as contacting the partially protected dsDNA with an exonuclease mixture. Alternatively, an exonuclease having AP endonuclease activity may perform both of these steps.

If the second strand of the partially protected dsDNA comprises a target sequence for a nicking endonuclease the methods may further comprise contacting the partially protected DNA with a nicking endonuclease prior to or at the same time as contacting the partially protected dsDNA with an exonuclease mixture.

The step of amplifying a DNA template may be an *in vitro* or *in vivo* amplification. Preferably, the amplification is an *in vitro* amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

The method may further comprise a step of purification of the digested precursor dsDNA after digesting the precursor dsDNA.

The method may further comprise a step of purification of the partially protected dsDNA after ligating the first and second adaptor molecules to the digested precursor dsDNA.

The method may further comprise a step of purification of the protected DNA after digesting the second strand of the partially protected dsDNA with an exonuclease mixture.

The method for producing a protected DNA comprising a single-stranded DNA (ssDNA) cassette may comprise:
(a) amplifying a DNA template to generate a precursor dsDNA, wherein the DNA template comprises a cassette and a Type IIS endonuclease target sequence 5' of the cassette and a Type IIS endonuclease target sequence 3' of the cassette, wherein the DNA template molecule is amplified by rolling circle amplification;
(b) contacting the precursor dsDNA comprising a first and a second strand with a Type IIS endonuclease, wherein the precursor dsDNA comprises on the first strand the cassette, the Type IIS endonuclease target sequence 5' of the cassette and the Type IIS endonuclease target sequence 3' of the cassette;
(c) digesting the precursor dsDNA with the Type IIS endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease mixture; and
(g) digesting the second strand of the partially protected dsDNA with the exonuclease mixture thereby generating the protected DNA; optionally wherein steps (b) (i.e. contacting the precursor dsDNA comprising a first and a second strand with an endonuclease) to (e) (i.e. ligating the first and second adaptor molecules to the digested precursor dsDNA) are performed in a single contiguous aqueous volume.

Producing protected DNA which is partially double-stranded may involve a partially protected dsDNA with a nuclease-resistant nucleotide in the second strand. Accordingly, it may be necessary to nick the second strand of the partially protected dsDNA to provide an unprotected 3' and/or 5' end nucleotide for the exonuclease. The method for producing a protected DNA comprising a single-stranded DNA (ssDNA) cassette may comprise:
(a) providing a partially protected double-stranded DNA (dsDNA) comprising a first strand and a second strand, wherein the first strand of the partially protected dsDNA comprises a cassette with x nuclease-resistant nucleotides at the 5' end of the cassette or 5' of the cassette, and y nuclease-resistant nucleotides at the 3'-end of the cassette or 3' of the cassette, wherein the second strand of the partially protected dsDNA comprises a sequence complementary to the cassette, y' nuclease-resistant nucleotides at the 5' end of the sequence complementary to the cassette or 5' of the sequence complementary to the cassette, and x' nuclease-resistant nucleotides at the 3'-end of the sequence complementary to the cassette or 3' of the sequence complementary to the cassette, wherein x is at least 1, x' is at least 1, y is at least 1 and y' is at least 1;
(b) nicking the second strand of the partially protected dsDNA between the x' and/or y' nuclease-resistant nucleotides; and
(c) digesting the second strand of the partially protected dsDNA with an exonuclease mixture thereby generating the protected DNA.

Nicking may be performed by contacting the partially protected dsDNA with a nicking endonuclease. The nicking endonuclease may be Nt.BspQI, Nt.CviPII, Nt.BstNBI, Nb.BsrDI, Nb.Btsl, Nt.Alwl, Nb.BbvCI, Nb.Bsml, Nb.BssSI and/or Nt.BsmAI. The second strand of the partially protected dsDNA may comprise a target sequence for the nicking endonuclease. Nicking the second strand of the partially protected dsDNA may be performed prior to digesting the second strand of the partially protected dsDNA with an exonuclease mixture. Alternatively, nicking the second strand of the partially protected dsDNA may be performed at the same time as digesting the second strand of the partially protected dsDNA. Nicking the second strand of the partially protected dsDNA may be performed under the same conditions as digesting the second strand of the partially protected dsDNA or the same conditions as digesting the precursor dsDNA.

The invention therefore provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotide at the 5' end of the cassette or 5' of the cassette, and at least 5 nuclease-resistant nucleotides at the 3'-end of the cassette or 3' of the cassette and the second strand comprises a nicking endonuclease target sequence;
(b) contacting the partially protected dsDNA with a nicking endonuclease that recognises the target sequence; and
(c) digesting the second strand of the partially protected dsDNA with an exonuclease mixture thereby generating the protected DNA.

The method for producing a protected DNA comprising a single-stranded DNA (ssDNA) cassette may comprise:
(a) contacting a precursor dsDNA comprising a first and a second strand with a Type IIS endonuclease, wherein the precursor dsDNA comprises on the first strand a cassette, a Type IIS endonuclease target sequence 5' of the cassette and a Type IIS endonuclease target sequence 3' of the cassette;
(b) digesting the precursor dsDNA with the Type IIS endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1 and wherein the first and/or second adaptor molecule comprises a nicking endonuclease target sequence;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette and the second strand comprises a nicking endonuclease target sequence;
(e) contacting the partially protected dsDNA with an exonuclease mixture and a nicking endonuclease having the target sequence in the second strand; and
(f) nicking and digesting the second strand of the partially protected dsDNA with the nicking endonuclease and the exonuclease mixture, respectively, thereby generating the protected DNA; optionally wherein steps (a) (i.e. contacting a precursor dsDNA comprising a first and a second strand with a Type IIS endonuclease) to (d) (i.e. ligating the first and second adaptor molecules to the digested precursor dsDNA) are performed in a single contiguous aqueous volume.

The precursor dsDNA may be a product of amplification. Preferably, the amplification is rolling circle amplification.

Alternatively, the nicking may be performed using an enzyme having AP endonuclease activity. An AP endonuclease recognises sites within a DNA molecule that are which is an apurinic or apyrimidinic site in the DNA that does not have a purine or a pyrimidine, also known as abasic sites. An AP endonuclease will excise the abasic site leaving exposed a 3' hydroxyl group and/or a 5' phosphate that are then able to be utilised by exonuclease enzymes to initiate digestion. An abasic site may be introduced into the second strand of the partially protected dsDNA, e.g. by way of adaptor molecule ligation. Alternatively, an excisable nucleotide may be introduced into the second strand of the partially protected dsDNA molecule (e.g. by way of adaptor molecule ligation), and the partially protected dsDNA molecule contacted with a DNA glycosylase that recognises the excisable nucleotide such that it modifies the excisable nucleotide to become an abasic site.

As such, the invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette, and the second strand comprises an excisable nucleotide;
(b) optionally contacting the partially protected dsDNA with a DNA glycosylase that recognises the excisable nucleotide; and
(b) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotides at the 5' end of the cassette or 5' of the cassette, and at least 3 nuclease-resistant nucleotides at the 3'-end of the cassette or 3' of the cassette, and the second strand comprises at least 2 excisable nucleotides;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotide at the 5' end of the cassette or 5' of the cassette, and at least 5 nuclease-resistant nucleotides at the 3'-end of the cassette or 3' of the cassette and the second strand comprises at least 2 excisable nucleotides;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1 and the second strand comprises n excisable nucleotides, wherein n is at least 1;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.
x and y may be independently selected from at least 1, at least 2, at least 3, at least 4 and at least 5. x and y and n may be different or the same. X and y may both be at least 3. X and y may both be at least 5. X may be 3 and y be 1 and *vice versa.* X may be 3 and y be 5 and *vice versa.* n may be 1, 2, 3, 4, or 5.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotides at the 5' end of the cassette or 5' of the cassette, and at least 3 nuclease-resistant nucleotides at the 3'-end of the cassette or 3' of the cassette, and the second strand comprises an excisable nucleotide and at least 3 protected nucleotides;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotide at the 5' end of the cassette or 5' of the cassette, and at least 5 nuclease-resistant nucleotides at the 3'-end of the cassette or 3' of the cassette and the second strand comprises at least 2 excisable nucleotides and at least 3 protected nucleotides;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and the second strand comprises n excisable nucleotides, and x' protected nucleotides at the 5' end of the cassette or 5' of the cassette, and y' nuclease-resistant nucleotides at the 3'-end of the cassette or 3' of the cassette, wherein x is 0 or at least 1 and y is 0 or at least 1 and wherein n is at least 1;
(b) optionally contacting the partially protected dsDNA with a DNA glycosylase that recognises the excisable nucleotide; and
(c) digesting the second strand of the partially protected dsDNA with an exonuclease mixture thereby generating the protected DNA.
x and y may be independently selected from at least 1, at least 2, at least 3, at least 4 and at least 5. x and y and n may be different or the same. X and y may both be at least 3. X and y may both be at least 5. X may be 3 and y be 1 and *vice versa.* X may be 3 and y be 5 and *vice versa.* n may be 1, 2, 3, 4, or 5.

The excisable nucleotides may be the same or different.

The excisable nucleotide may be 8-oxoguanadine, 2,6-diamino-4-hydroxy-5-formamidopyrimidine (FapyG or FapyA), 3-methyladenine, 7-methylguanosine, hypoxanthine, xanthine or thymidine, uracil, thymine glycol, 5-hydroxycytosine (5-hC), 5-hydroxyuracil (5-hU), 3-methylguanine (3-meG), 3,N4-ethenocytosine or an ethylated base.

DNA glycosylases are able to recognise damaged bases (excisable nucleotides). They cleave the N-glycosidic bond of the damaged base, leaving an AP site. Different DNA glycosylases recognise different damaged (excisable) bases. The DNA glycosylase may be OGG1, MPG, SMUG1, UNG1, MBD4, TDG, MYH1, NTHL1, NEIL1, NEIL2 or NEIL3.

The second strand may comprise an abasic site, also known as an AP site, which is an apurinic or apyrimidinic site in the DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and the second strand comprises n abasic sites, and x' protected nucleotides at the 5' end of the cassette or 5' of the cassette, and y' nuclease-resistant nucleotides at the 3'-end of the cassette or 3' of the cassette, wherein x is 0 or at least 1 and y is 0 or at least 1 and wherein n is at least 1;
(b) optionally contacting the partially protected dsDNA with an AP endonuclease; and
(c) digesting the second strand of the partially protected dsDNA with an exonuclease mixture thereby generating the protected DNA.

AP endonucleases are able to cleave an abasic site (an AP site) to expose a 3' hydroxy group and/or a 5' phosphate group to render them available for exonuclease digestion. Alternatively, an exonuclease with AP endonuclease activity may perform both steps. The AP endonuclease may be a Class I AP endonuclease, a Class II AP endonuclease, a Class III AP endonuclease, and/or a Class IV AP endonuclease. Class I AP endonucleases (EC 4.2.99.18) cleave 3' to AP sites by a β-lyase mechanism, leaving an unsaturated aldehyde, termed a 3'-(4-hydroxy-5-phospho-2-pentenal) residue, and a 5'-phosphate. Class II AP endonucleases incise DNA 5' to AP sites by a hydrolytic mechanism, leaving a 3'-hydroxyl and a 5'-deoxyribose phosphate residue. Class III and class IV AP endonucleases also cleave DNA at the phosphate groups 3' and 5' to the baseless site, but they generate a 3'-phosphate and a 5'-OH.

Thus, the invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1 and the second strand comprises n abasic sites, wherein n is at least 1; and
(b) contacting the second strand of the partially protected dsDNA with an exonuclease mixture comprising an exonuclease having AP endonuclease activity, thereby nicking and digesting the second strand and thereby generating the protected DNA.
x and y may be independently selected from at least 1, at least 2, at least 3, at least 4 and at least 5. x and y and n may be different or the same. X and y may both be at least 3. X and y may both be at least 5. X may be 3 and y be 1 and *vice versa.* X may be 3 and y be 5 and *vice versa.* n may be 1, 2, 3, 4, or 5.

The method may be a cell-free method.

Aspects of the invention described above in relation to methods for producing protected DNA apply *mutatis mutandis* to all aspects of the invention described herein.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises a nuclease resistant nucleotide and the second adaptor molecule comprises a nuclease-resistant nucleotide and the first and/or second adaptor molecule comprises an excisable nucleotide; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 3 nuclease resistant nucleotides and the second adaptor molecule comprises at least 3 nuclease-resistant nucleotides and the first and/or second adaptor molecule comprises an excisable nucleotide or an abasic site; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 5 nuclease resistant nucleotides and the second adaptor molecule comprises at least 5 nuclease-resistant nucleotides and the first and/or second adaptor molecule comprises an excisable nucleotide or an abasic site; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1 and the first and/or second adaptor molecule comprises n excisable nucleotides wherein n is at least 1; and
(d) ligating the first adaptor molecule to a first end of the digested precursor dsDNA and ligating the second adaptor molecule to a second end of the digested precursor dsDNA thereby generating the partially protected dsDNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise a nuclease-resistant nucleotide and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette and the second strand comprises an excisable nucleotide;
(e) contacting the partially protected dsDNA with a DNA glycosylase and an exonuclease mixture; and
(f) incubating the partially protected dsDNA with the DNA glycosylase and the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 3 nuclease-resistant nucleotides and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette and wherein the second strand comprises an excisable nucleotide (from the first and/or second adaptor molecule);
(e) contacting the partially protected dsDNA with a DNA glycosylase and an exonuclease mixture; and
(f) incubating the partially protected dsDNA with the DNA glycosylase and the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 5 nuclease-resistant nucleotides and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette and the second strand comprises at least 2 excisable nucleotides (from the first and/or second adaptor molecule);
(e) contacting the partially protected dsDNA with a DNA glycosylase and an exonuclease mixture; and
(f) incubating the partially protected dsDNA with the DNA glycosylase and the exonuclease mixture thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and n excisable nucleotides and the second adaptor molecule comprises y nuclease-resistant nucleotides and m excisable nucleotides, and wherein x is at least 1 and y is at least 1, wherein n is 0 or at least 1 and m is 0 or at least 1 and n+m is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette and the second strand comprises n+m excisable nucleotides;
(e) contacting the partially protected dsDNA with a DNA glycosylase and an exonuclease mixture; and
(f) incubating the partially protected dsDNA with the DNA glycosylase and the exonuclease mixture thereby generating the protected DNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1 and the first and/or second adaptor molecule comprises n abasic sites wherein n is at least 1; and
(d) ligating the first adaptor molecule to a first end of the digested precursor dsDNA and ligating the second adaptor molecule to a second end of the digested precursor dsDNA thereby generating the partially protected dsDNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and n abasic sites and the second adaptor molecule comprises y nuclease-resistant nucleotides and m abasic sites, and wherein x is at least 1 and y is at least 1, wherein n is 0 or at least 1 and m is 0 or at least 1 and n+m is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette and the second strand comprises n+m abasic sites (from the first and/or second adaptor molecules);
(e) contacting the partially protected dsDNA with an exonuclease mixture and optionally with an AP endonuclease; and
(f) incubating the partially protected dsDNA with the exonuclease mixture and the optional AP endonuclease thereby generating the protected DNA.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3!,Sap!, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV11, BstV2!, Bsul, BtgZI, BtsCI, Btsl-v2, BtsMutl, Bve!, Csel, CspCI, Eam1104I, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269!, NmeAIII, PaqCI, PciSI, Pctl, PleI, Ppsl, Psrl, Schl, SfaNI, Taqll, TspDTI and/or TspGWI restriction enzyme.

Type IIS restriction endonucleases cleave dsDNA outside of the recognition sequence (i.e. an endonuclease target sequence), which means that the recognition sequence (i.e. endonuclease target sequence) is not included in the cleaved product.

The ligase may be a DNA ligase, such as a T4 DNA ligase, T7 DNA ligase, mammalian DNA ligase I, III and IV; Taq DNA ligase, Tth DNA ligase, or *E. coli* DNA ligase. The ligase is preferably T7 DNA ligase.

The DNA glycosylase may be OGG1, MPG, SMUG1, UNG1, MBD4, TDG, MYH1, NTHL1, NEIL1, NEIL2 or NEIL3.

The exonuclease may be an exonuclease which acts on single-stranded DNA (e.g. *E.* coliexonuclease I, exonuclease VII, exonuclease T, RecJf, RecJ). This may be the case when the partially protected dsDNA is denatured.

Alternatively or in combination, the exonuclease may be an exonuclease which acts on double-stranded DNA (e.g. *E.* coli exonuclease III, T7 exonuclease, exonuclease V, exonuclease VIII, T5 exonuclease, lambda exonuclease). This may be the case when the partially protected dsDNA is not denatured.

The protected DNA may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease I, exonuclease T, exonuclease V and/or exonuclease VII) and/or 5'-end exonuclease digestion (e.g. by exonuclease VIII, RecJf, RecJ, T7 exonuclease, Lambda exonuclease, T5 exonuclease, exonuclease V and/or exonuclease VII).

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to exonuclease digestion. For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

As used herein "excisable nucleotide" means a nucleotide that is recognised to be a mismatch or damaged by DNA repair enzymes, such that the site of the DNA molecule may be modified and/or nicked to expose a free 3' or 5' hydroxyl, such that an exonuclease mixture is able to digest the DNA. An excisable nucleotide may be an oxidised base, such as 8-oxoguanadine, 2,6-diamino-4-hydroxy-5-formamidopyrimidine (FapyG or FapyA); an alkylated base, such as 3-methyladenine, 7-methylguanosine; a deaminated base, such as hypoxanthine, xanthine or thymidine; uracil.

The step of digesting the precursor dsDNA with the endonuclease to generate a digested precursor dsDNA may be performed by incubating the combined components under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of ligating the first and second adaptor molecules to the digested precursor dsDNA may be performed by incubating the combined components under conditions that promote ligation of the first and second adaptor molecules to the digested precursor dsDNA to produce the partially protected dsDNA. The ligating may be performed by creating a covalent link between the first and/or second adaptor molecule and the first and/or second end of the digested precursor dsDNA. Preferably, the step of ligation is performed in the presence of T7 DNA ligase.

The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. Preferably, the ligation is at least 50%, or at least 70% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, at least 25 % or at least 30% of the digested precursor dsDNA is incorporated into partially protected dsDNA

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the precursor dsDNA generated by the amplification (e.g. rolling circle amplification) is first quantified so that the amount of the precursor dsDNA used as the starting material during the digestion/ligation reaction is known. After all the enzymatic reactions, the partially protected dsDNA is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligating the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

Using the above conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E. coli* DNA ligase. Preferably, the ligase is T7 DNA ligase.

The step of digesting the second strand of the partially protected dsDNA with an exonuclease mixture may be performed at a temperature of 5-90°C, 10-80°C, 15-70°C, 20-60°C, 25-50°C, 30-45°C or 35-40°C. Preferably, the step of digesting the second strand of the partially protected dsDNA with an exonuclease mixture is performed at a temperature of 30-45°C. The step of digesting the second strand of the partially protected dsDNA with an exonuclease mixture may be performed for at least 10, at least 20, at least 30, at least 40, at least 50, at least 60 or at least 120 minutes. Preferably, the step of digesting the second strand of the partially protected dsDNA with an exonuclease mixture may be performed for at least 60 minutes. For example, the step of digesting the second strand of the partially protected dsDNA with an exonuclease mixture may be performed at around 37 °C for at least 120 minutes.

The step of digesting the second strand of the partially protected dsDNA with an exonuclease mixture may be followed by inactivating the exonucleases in the exonuclease mixture, for example by heat-inactivation. Thus, the step of inactivating the exonucleases in the exonuclease mixture may be performed at a temperature of 60-90°C, 65-85°C or 70-80°C. Preferably, the step of inactivating the exonucleases in the exonuclease mixture is performed at a temperature of 70-80°C. The step of inactivating the exonucleases in the exonuclease mixture may be performed at a temperature of at least 60°C, at least 65°C, at least 70°C or at least 80°C. Preferably, the step of inactivating the exonucleases in the exonuclease mixture is performed at a temperature of at least 70°C. The step of inactivating the exonucleases in the exonuclease mixture may be performed for at least 1, at least 5, at least 10, at least 20 or at least 30 minutes. Preferably, the step of inactivating the exonucleases in the exonuclease mixture is performed for at least 5 minutes.

The step of digesting the second strand of the partially protected dsDNA with an exonuclease mixture may be performed at a temperature of 30-45°C for at least 60 minutes followed by performing the step of inactivating the exonucleases in the exonuclease mixture at a temperature of at least 70°C for at least 5 minutes.

The step of incubating the partially protected dsDNA with a DNA glycosylase and an exonuclease mixture may be performed at a temperature of 5-90°C, 10-80°C, 15-70°C, 20-60°C, 25-50°C, 30-45°C or 35-40°C. Preferably, the step of incubating the partially protected dsDNA with a DNA glycosylase and an exonuclease mixture is performed at a temperature of 30-45°C. The step of incubating the partially protected dsDNA with a DNA glycosylase and an exonuclease mixture may be performed for at least 10, at least 20, at least 30, at least 40, at least 50, at least 60 or at least 120 minutes. Preferably, the step of incubating the partially protected dsDNA with a DNA glycosylase and an exonuclease mixture may be performed for at least 60 minutes. For example, incubating the partially protected dsDNA with a DNA glycosylase and an exonuclease mixture may be performed at around 37 °C for at least 120 minutes.

The step of incubating the partially protected dsDNA with a DNA glycosylase and an exonuclease mixture may be followed by inactivating the DNA glycosylase and the exonucleases, for example by heat-inactivation. Thus, the step of inactivating the DNA glycosylase and the exonucleases may be performed at a temperature of 60-100°C, 80-100°C, 95-100°C, 60-90°C, 65-85°C or 70-80°C. Preferably, the step of inactivating the DNA glycosylase and the exonucleases is performed at a temperature of 70-80°C or 95-100°C. The step of inactivating the DNA glycosylase and the exonucleases may be performed at a temperature of at least 60°C, at least 65°C, at least 70°C, at least 80°C, at least 90°C, at least 95°C, or at least 97°C. Preferably, the step of inactivating the DNA glycosylase and the exonucleases is performed at a temperature of at least 70°C or at least 97°C. The step of inactivating the DNA glycosylase and the exonucleases may be performed for at least 1, at least 5, at least 10, at least 20 or at least 30 minutes. Preferably, the step of inactivating the DNA glycosylase and the exonucleases is performed for at least 5 minutes.

The step of incubating the partially protected dsDNA with a DNA glycosylase and an exonuclease mixture may be performed at a temperature of 30-45°C for at least 60 minutes followed by performing the step of inactivating the exonucleases in the exonuclease mixture at a temperature of at least 70°C for at least 5 minutes.

Incubating with the DNA glycosylase may be performed prior to digesting the second strand of the partially protected dsDNA with an exonuclease mixture. Alternatively, incubating the partially protected dsDNA with the DNA glycosylase may be performed at the same time as digesting the second strand of the partially protected dsDNA.

Aspects of the invention described above in relation to methods for producing protected DNA apply *mutatis mutandis* to all aspects of the invention described herein.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may be a single cassette. The term "single cassette" as used herein is intended to encompass a product that does not comprise or consist of a plurality of cassettes. That is to say that the protected DNA (or partially protected dsDNA or precursor dsDNA) comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may comprise a coding sequence. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000 nucleotides. Preferably, the gene comprises at least 200 nucleotides.

The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The first and second adaptor molecules may be nucleic acids, optionally DNA. The first and second adaptor molecules may comprise a first strand hybridised to a second strand (e.g. dsDNA).

The first and second adaptor molecules may have different sequences.

The first and second adaptor molecules may comprise dsDNA comprising a first strand and a second strand. The first strands of the first and second adaptor molecules may be ligated by the ligase to the first strand of the digested precursor dsDNA molecule and the second strands of the first and second adaptor molecules may be ligated by the ligase to the second strand of the digested precursor dsDNA molecule.

The first adaptor molecule may be compatible with the first end of the digested precursor dsDNA and incompatible with the second end of the digested precursor dsDNA. The second adaptor molecule may be compatible with the second end of the digested precursor dsDNA and incompatible with the first end of the digested precursor dsDNA.

The first adaptor molecule may be compatible with the 5' end of the first strand of the digested precursor dsDNA and incompatible with the 3' end of the first strand of the digested precursor dsDNA. The second adaptor molecule may be compatible with the 3' end of the first strand of the digested precursor dsDNA and incompatible with the 5' end of the first strand of the digested precursor dsDNA.

The first strand of the first adaptor molecule may be compatible with the 5' end of the first strand of the digested precursor dsDNA and incompatible with the 3' end of the first strand and the 3' and 5' ends of the second strand of the digested precursor dsDNA. The second strand of the first adaptor molecule may be compatible with the 3' end of the second strand of the digested precursor dsDNA and incompatible with the 5' end of the second strand and the 3' and 5' ends of the first strand of the digested precursor dsDNA.

The first strand of the second adaptor molecule may be compatible with the 3' end of the first strand of the digested precursor dsDNA and incompatible with the 5' end of the first strand and the 3' and 5' ends of the second strand of the digested precursor dsDNA. The second strand of the second adaptor molecule may be compatible with the 5' end of the second strand of the digested precursor dsDNA and incompatible with the 3' end of the second strand and the 3' and 5' ends of the first strand of the digested precursor dsDNA.

The second strand of the first and second adaptor molecules may not be resistant to exonuclease digestion. The second strand of the first and second adaptor molecules may be susceptible to exonuclease digestion. The second strand of the first and second adaptor molecules may not comprise any nuclease-resistant nucleotides. The second strand may comprise n excisable nucleotides. The n excisable nucleotides are preferably the same. The second strand may comprise n excisable nucleotides and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or 3' of the cassette. The n excisable nucleotides are preferably at the same end of cassette as the protected nucleotides, i.e. the excisable nucleotide and the protected nucleotides are 5' of or at the 5' end of the cassette, or the excisable nucleotides and the protected nucleotides are 3' of or at the 3' end of the cassette. There may be at least 1, at least 2, at least 3, at least 4, at least 5, at least 6, at least 7 or at least 8 nucleotides between the protected nucleotides and the excisable nucleotides. The protected nucleotides are preferably to the 3' of the excisable nucleotides when the protected nucleotides and the excisable nucleotides are at the 3' end of or 3' of the cassette. The protected nucleotides are preferably to the 5' of the excisable nucleotides when the protected nucleotides and the excisable nucleotides are at the 5' end of or 5' of the cassette.

The second strand may comprise n abasic sites. The second strand may comprise n abasic sites and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or 3' of the cassette. The n abasic sites are preferably at the same end of cassette as the protected nucleotides, i.e. the abasic sites and the protected nucleotides are 5' of or at the 5' end of the cassette, or the abasic sites and the protected nucleotides are 3' of or at the 3' end of the cassette. There may be at least 1, at least 2, at least 3, at least 4, at least 5, at least 6, at least 7 or at least 8 nucleotides between the protected nucleotides and the abasic sites. The protected nucleotides are preferably to the 3' of the abasic sites when the protected nucleotides and the abasic sites are at the 3' end of or 3' of the cassette. The protected nucleotides are preferably to the 5' of the excisable nucleotides when the protected nucleotides and the abasic sites are at the 5' end of or 5' of the cassette.

The second strand may comprise a nicking endonuclease target sequence. The second strand may comprise a nicking endonuclease target sequence and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or 3' of the cassette. The nicking endonuclease target sequence is preferably at the same end of cassette as the protected nucleotides, i.e. the nicking endonuclease target sequence and the protected nucleotides are 5' of or at the 5' end of the cassette, or the nicking endonuclease target sequence and the protected nucleotides are 3' of or at the 3' end of the cassette. There may be at least 1, at least 2, at least 3, at least 4, at least 5, at least 6, at least 7 or at least 8 nucleotides between the protected nucleotides and the nicking endonuclease target sequence. The protected nucleotides are preferably to the 3' of the nicking endonuclease target sequence when the protected nucleotides and the nicking endonuclease target sequence are at the 3' end of or 3' of the cassette. The protected nucleotides are preferably to the 5' of the nicking endonuclease target sequence when the protected nucleotides and the nicking endonuclease target sequence are at the 5' end of or 5' of the cassette.

The first strand of the first adaptor molecule may comprise the x nucleotide-resistant nucleotides and the first strand of the second adaptor molecule may comprise the y nucleotide-resistant nucleotides. The nucleotide-resistant nucleotide(s) in the first strand of the first adaptor molecule may be located at the 5' or 3' end, preferably the 5' end; and/or the nucleotide-resistant nucleotide(s) in the first strand of the second adaptor molecule may be located at the 5' or 3' end, preferably the 3' end. The nuclease-resistant nucleotide may be a phosphorothioated nucleotide.

The second strand of the first adaptor molecule may comprise the x' nucleotide-resistant nucleotides and the second strand of the second adaptor molecule may comprise the y' nucleotide-resistant nucleotides. The nucleotide-resistant nucleotide(s) in the second strand of the first adaptor molecule may be located at the 5' or 3' end, preferably the 3' end; and/or the nucleotide-resistant nucleotide(s) in the second strand of the second adaptor molecule may be located at the 5' or 3' end, preferably the 5' end. The nuclease-resistant nucleotide may be a phosphorothioated nucleotide.

The first adaptor molecule and/or the second adaptor molecule may be a synthetic adaptor molecule.

The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a first strand and a second strand, wherein the first strand and the second strand are linked together in a hairpin such that the first strand is hybridized to the second strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the digested precursor dsDNA may comprise a 3' or a 5' overhang. A portion of the first adaptor molecule (e.g. the overhang) may be complementary to the first end of the digested precursor dsDNA. A portion of the second adaptor molecule may be complementary to the second end of the digested precursor dsDNA.

If the first and/or second adaptor molecules comprise a hairpin, following ligation of the first and second adaptor molecules to the digested precursor dsDNA there may be a hairpin 3' and/or 5' of the cassette. The hairpin may link the first and second strands of the digested precursor dsDNA to form a closed loop at one or both ends. Thus, the methods described herein may further comprise nicking a closed loop at one or both ends in order to generate a partially protected dsDNA with a 3' and/or 5' end nucleotide on the second strand.

The first adaptor molecule and/or the second adaptor molecule may comprise an overhang. The first and/or second ends of the digested precursor dsDNA may comprise a 3' or a 5' overhang. A portion of the first adaptor molecule (e.g. the overhang) may be complementary to the first end of the digested precursor dsDNA. A portion of the second adaptor molecule (e.g. the overhang) may be complementary to the second end of the digested precursor dsDNA.

The first adaptor molecule may comprise an overhang which is complementary to the first end of the digested precursor dsDNA. The second adaptor molecule may comprise an overhang which is complementary to the second end of the digested precursor dsDNA.

The first adaptor molecule may comprise an overhang which is non-complementary to the second end of the digested precursor dsDNA. The second adaptor molecule may comprise an overhang which is non-complementary to the first end of the digested precursor dsDNA.

The first adaptor molecule may comprise an overhang complementary to an overhang at the first end of the digested precursor dsDNA and the second adaptor molecule may comprise an overhang complementary to an overhang at the second end of the digested precursor dsDNA. Optionally, the overhang of the first adaptor molecule may not be complementary to the overhang at the second end of the digested precursor dsDNA and the overhang of the second adaptor molecule may not be complementary to the overhang at the first end of the digested precursor dsDNA.

The first adaptor molecule may comprise an overhang that is complementary to and anneals to a 5' overhang of the digested precursor dsDNA and the second adaptor molecule may comprise an overhang that is complementary to and anneals to a 3' overhang of the digested precursor dsDNA.

The first adaptor molecule may comprise an overhang that is complementary to and anneals to a 3' overhang of the digested precursor dsDNA and the second adaptor molecule may comprise an overhang that is complementary to and anneals to a 5' overhang of the digested precursor dsDNA.

The first adaptor molecule and/or the second adaptor molecule may not be a plasmid or a vector DNA.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the digested precursor dsDNA (which may comprise a 3'-OH group at first and/or second ends). The first adaptor molecule and/or the second adaptor molecule may comprise a 3'-OH. The 3'-OH may facilitate ligation to the digested precursor dsDNA (which may comprise a 5' phosphate at first and/or second ends).

The first strand of the first adaptor molecule may comprise the sequence of SEQ ID NO: 7 or a portion thereof. The second strand of the first adaptor molecule may comprise the sequence of SEQ ID NO: 8 or 9 or a portion thereof. The first strand of the second adaptor molecule may comprise the sequence of SEQ ID NO: 10 or a portion thereof. The second strand of the second adaptor molecule may comprise the sequence of SEQ ID NO: 11 or 12 or a portion thereof. The first and second adapter molecules may comprise a different nucleic acid sequence. The first strand of the first adaptor molecule may comprise the sequence of SEQ ID NO: 1 or a portion thereof. The second strand of the first adaptor molecule may comprise the sequence of SEQ ID NO: 2 or a portion thereof. The first strand of the second adaptor molecule may comprise the sequence of SEQ ID NO: 3 or a portion thereof. The second strand of the second adaptor molecule may comprise the sequence of SEQ ID NO: 4 or a portion thereof. The first strand of the first adaptor molecule may comprise the sequence of SEQ ID NO: 16 or a portion thereof. The second strand of the first adaptor molecule may comprise the sequence of SEQ ID NO: 17 or a portion thereof. The first strand of the second adaptor molecule may comprise the sequence of SEQ ID NO: 18 or a portion thereof. The second strand of the second adaptor molecule may comprise the sequence of SEQ ID NO: 19 or a portion thereof.

The first adaptor molecule may comprise a portion that is complementary to the first end of the digested precursor dsDNA. The second adaptor molecule may comprise a portion that is complementary to the second end of the digested precursor dsDNA. The first adaptor molecule may comprise a portion that anneals to the first end of the digested precursor dsDNA. The second adaptor molecule may comprise a portion that anneals to the second end of digested precursor dsDNA. The first adaptor molecule may comprise a portion that is complementary and anneals to the first end of the digested precursor dsDNA. The second adaptor molecule may comprise a portion that is complementary and anneals to the second end of the digested precursor dsDNA.

The portion that is complementary or anneals to the first or second end of the digested precursor dsDNA may be a 5' overhang or a 3' overhang of the first and/or second adaptor molecule. The overhang of the first adaptor molecule may be complementary to the first end of the digested precursor dsDNA and/or the overhang of the second adaptor molecule may be complementary to the second end of the digested precursor dsDNA. The overhang of the first adaptor molecule may anneal to the first end of the digested precursor dsDNA and/or the overhang of the second adaptor molecule may anneal to the second end of the digested precursor dsDNA. The overhang of the first adaptor molecule may be complementary to and anneal to the first end of the digested precursor dsDNA and/or the overhang of the second adaptor molecule may be complementary to and anneal to the second end of the digested precursor dsDNA.

The first adaptor molecule may be appended to a first end of the digested precursor dsDNA and the second adaptor molecule may be appended to a second end of the digested precursor dsDNA. The appending of the first adaptor molecule and/or the second adaptor molecule may be performed by hybridization or ligation of the adaptor molecules to the ends of the digested precursor dsDNA. Thus, the first adaptor molecule may be hybridized to the first end of the digested precursor dsDNA. The second adaptor molecule may be hybridized to the second end of the digested precursor dsDNA. The first adaptor molecule may be ligated to the first end of the digested precursor dsDNA. The second adaptor molecule may be ligated to the second end of the digested precursor dsDNA. The appending of the first adaptor molecule and the second adaptor molecule may be performed by both hybridization and ligation of the adaptor molecules to the ends of the digested precursor dsDNA. Thus, the first adaptor molecule may be hybridized and ligated to the first end of the digested precursor dsDNA. The second adaptor molecule may be hybridized and ligated to the second end of the digested precursor dsDNA. The hybridization is based on complementarity of a portion of the first and/or second adaptor molecules to the first and/or second end of the digested precursor dsDNA.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQI, BtgZI, Esp3I, SapI, Aarl, Acc361, AclWI, Acul, Ajul, Alol, Alw261, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV1I, BstV2!, Bsul, BtgZI, BtsCI, Btsl-v2, BtsMutl, Bve!, Csel, CspCI, Eam11 04!, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269!, NmeAIII, PaqCl, PciSI, Pctl, Plel, Ppsl, Psrl, Schl, SfaNI, Taqll, TspDTI and/or TspGWI target sequences.

The first adaptor molecule and/or the second adaptor molecule may comprise one or more locked nucleic acids (LNAs).

The first adaptor molecule and/or the second adaptor molecule may confer resistance to the nuclease digestion, such as exonuclease digestion (e.g. exonuclease III or VIII digestion).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The first and second adaptor molecules may comprise one or more phosphorothioated nucleotides, such that, once the adaptor molecules are appended (e.g. ligated) to the digested precursor dsDNA and the second strand of the partially protected dsDNA is digested by the exonuclease mixture, the protected DNA is resistant to nuclease digestion or has improved or enhanced resistance to nuclease digestion. The protected DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand. The first and/or second adaptor molecule may comprise protected nucleotides in the first strand and may not comprise protected nucleotides in the second strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the digested precursor dsDNA and the partially protected dsDNA is digested by the exonuclease mixture, the protected DNA may comprise a protected nucleotide at the 5' end of the ssDNA cassette or 5' of the ssDNA cassette, and a nuclease-resistant nucleotide at the 3'-end of the ssDNA cassette or 3' of the ssDNA cassette.

The nucleotides resistant to exonuclease digestion (i.e. protected nucleotides) suitable for use in the present invention may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(a-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The first and/or second adaptor molecules may comprise at least 1 excisable nucleotide on the second strand. The first and/or second adaptor molecule may comprise at least 2 excisable nucleotides on the second strand. The first and second adaptor molecules may not comprise excisable nucleotides on the first strand. Preferably the at least 2 excisable nucleotides are the same. The excisable nucleotides may be 5' of any protected nucleotides, when the protected nucleotides are at the 3' end or 3' of the cassette. There may be at least 1, at least 2, at least 3, at least 4, at least 5, at least 6, at least 7 or at least 8 nucleotides between the protected nucleotides and the excisable nucleotides. The excisable nucleotides may be 3' of any protected nucleotides, when the protected nucleotides are at the 5' end or 5' of the cassette. There may be at least 1, at least 2, at least 3, at least 4, at least 5, at least 6, at least 7 or at least 8 nucleotides between the protected nucleotides and the excisable nucleotides.

The excisable nucleotide may be 8-oxoguanadine, 2,6-diamino-4-hydroxy-5-formamidopyrimidine (FapyG or FapyA), 3-methyladenine, 7-methylguanosine, hypoxanthine, xanthine or thymidine, uracil, thymine glycol, 5-hydroxycytosine (5-hC), 5-hydroxyuracil (5-hU), 3-methylguanine (3-meG), 3,N4-ethenocytosine or an ethylated base.

The first and/or second adaptor molecules may comprise at least 1 abasic site on the second strand. The first and/or second adaptor molecule may comprise at least 2 abasic sites on the second strand. The first and second adaptor molecules may not comprise abasic sites on the first strand. The abasic sites may be 5' of any protected nucleotides, when the protected nucleotides are at the 3' end or 3' of the cassette. There may be at least 1, at least 2, at least 3, at least 4, at least 5, at least 6, at least 7 or at least 8 nucleotides between the protected nucleotides and the abasic sites. The abasic sites may be 3' of any protected nucleotides, when the protected nucleotides are at the 5' end or 5' of the cassette. There may be at least 1, at least 2, at least 3, at least 4, at least 5, at least 6, at least 7 or at least 8 nucleotides between the protected nucleotides and the abasic sites.

Alternatively or additionally, the first and/or second adaptor molecule may comprise a target sequence for a nicking endonuclease. The nicking endonuclease may be Nt.BspQI, Nt.CviPII, Nt.BstNBI, Nb.BsrDI, Nb.BtsI, Nt.Alwl, Nb.BbvCl, Nt.BbvCl, Nb.Bsml, Nb.BssSI and/or Nt.BsmAl. The second strand of the partially protected dsDNA (by way of the ligation of the first and/or second adaptor molecule) may comprise a target sequence for the nicking endonuclease.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probetarget base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the protected DNA described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the protected DNA. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the protected DNA product, the functional portion may comprise a fluorophore, a radioactive compound or a barcode.

A signal corresponding to the presence, absence and/or level of the protected DNA may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the protected DNA. The binding moiety is capable of binding to 3' and/or 5' end of the protected DNA. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the protected DNA and the second binding moiety linked to the second barcoded portion may bind to the 5' end of the protected DNA. The 3' and 5' ends may comprise a 3' overhang, a 5' overhang or a blunt end.

A signal corresponding to the presence, absence and/or level of the protected DNA may be measured using a fluorophore (i.e. a fluorescently-labelled molecule), which is attached or bound to the protected DNA. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an aptamer.

The first adaptor molecule and/or the second adaptor molecule may be a synthetic adaptor molecule.

The first adaptor molecule and/or the second adaptor molecule may not be a plasmid or a vector DNA.

The first adaptor molecule and/or the second adaptor molecule may comprise a single-stranded portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the digested precursor dsDNA.

The first adaptor molecule may comprise a portion that is complementary to the first end of digested precursor dsDNA. The second adaptor molecule may comprise a portion that is complementary to the second end of the digested precursor dsDNA. The first adaptor molecule may comprise a portion that anneals to the first end of the digested precursor dsDNA. The second adaptor molecule may comprise a portion that anneals to the second end of the digested precursor dsDNA.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end. The overhang may have at least 3 nucleotides (preferably from 4 to 6 nucleotides). The overhang of the first adaptor molecule and/or the second adaptor molecule may be complementary to the first and/or second end of the digested precursor dsDNA. The overhang of the first adaptor molecule and/or the second adaptor molecule may anneal to the first and/or second end of the digested precursor dsDNA.

The partially protected dsDNA may be generated from the precursor dsDNA by performing the steps described herein in a single reaction (i.e. as a single step) in a single contiguous aqueous volume. For example, the steps of contacting a precursor dsDNA with an endonuclease, digesting the precursor dsDNA with the endonuclease to generate a digested precursor dsDNA, contacting the digested precursor dsDNA with a ligase and first and second adaptor molecules, and ligating the first and second adaptor molecules to the digested precursor DNA thereby generating the partially protected dsDNA, may be performed in a single reaction (i.e. as a single step) in a single contiguous aqueous volume. Thus, the partially protected dsDNA may be generated by incubating the precursor dsDNA with the endonuclease, the ligase and first and second adaptor molecules in a single reaction (i.e. a single step) in a single contiguous aqueous volume. The single contiguous aqueous volume may be incubated in order to allow the required digestion and ligation.

The single contiguous aqueous volume may be incubated to generate the partially protected dsDNA by digesting the precursor dsDNA and ligating the first and second adaptor molecules to the digested precursor dsDNA.

The step of incubating the single contiguous aqueous volume may be performed under conditions that promote ligation of the first and second adaptor molecules to the digested precursor dsDNA to produce the partially protected dsDNA. The appending may be performed by creating a covalent link between the first and/or second adaptor molecule and the end(s) of the digested precursor dsDNA.

The step of incubating the single contiguous aqueous volume may be performed under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the digested precursor dsDNA to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75%, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, the ligation is at least 15% efficient.

The step of ligation of the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The DNA template may comprise at least one cleavable target sequence. The cleavable target sequence may be an endonuclease target sequence. Thus, the DNA template may comprise at least one endonuclease target sequence. Preferably, the DNA template comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQI, BtgZI, Esp3!,Sap!, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAl, Bful, Bmrl, Bmsl, Bmul, Bpil, Bpml, BpuEI, BsaXI, Bse11, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV11, BstV2!, Bsul, BtgZI, BtsCI, Btsl-v2, BtsMutl, Bve!, Csel, CspCI, Eam1104!, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, MlyI, Mmel, MnlI, Mva1269I, NmeAIII, PaqCl, PciSI, Pctl, Plel, Ppsl, Psrl, Schl, SfaNI, Taqll, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the protected DNA.

The DNA template used in the methods described herein may be single-stranded or double-stranded. Preferably, the DNA template is double-stranded. The DNA template may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template may be (i) a circular DNA molecule obtained from recombinase reaction, preferably Cre recombinase reaction, or (ii) a circular DNA molecule obtained from ligase reaction, preferably using the golden gate assembly. The DNA template may be an enzymatically produced covalently-closed linear DNA molecule. For example, the DNA template may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the DNA template may comprise a double-stranded DNA and a single-stranded hairpin loop.

The DNA template may be linear. If the DNA template is linear, prior to amplification (e.g. rolling circle amplification), a DNA template may be circularized to produce a DNA template suitable for use in the methods described herein.

The DNA template may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the protected DNA, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The digested precursor dsDNA may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the digested precursor dsDNA is at least 200 base pairs long.

The first end of the digested precursor dsDNA may be complementary to a portion of the first adaptor molecule. The second end of the digested precursor dsDNA may be complementary to a portion of the second adaptor molecule. The first end and/or the second end of the digested precursor dsDNA may be generated by endonuclease digestion.

The digested precursor dsDNA may comprise a 3'-OH group at first and/or second ends. The 3'-OH group may facilitate ligation to the first and/or second adaptor molecule(s) (which may comprise a 5' phosphate). The digested precursor dsDNA may comprise a 5' phosphate at first and/or second ends. The 5' phosphate may facilitate ligation to the first and/or second adaptor molecule(s) (which may comprise a 3'-OH group).

The digested precursor dsDNA may comprise an overhang. For example, the digested precursor dsDNA may comprise a 5' overhang or a 3' overhang. The digested precursor dsDNA may comprise a blunt end or blunt ends. The digested precursor dsDNA may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the first or second strand of the digested precursor dsDNA.

### 4. Kits

The invention provides a kit comprising components required to carry out the methods described herein. The kit may comprise at least:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1;
(b) an endonuclease;
(c) a ligase; and
(d) an exonuclease mixture.

The exonuclease mixture may comprise at least a 5' to 3' exonuclease and a 3' to 5' exonuclease. The exonuclease mixture may comprise at least three exonucleases, wherein at least one of the at least three exonucleases is a 5' to 3' exonuclease and at least one of the at least three exonucleases is a 3' to 5' exonuclease. The exonuclease mixture may comprise at least three exonucleases, wherein at least two of the at least three exonucleases are 5' to 3' exonucleases and at least one of the at least three exonucleases is a 3' to 5' exonuclease. The exonuclease mixture may comprise at least three exonucleases, wherein at least one of the at least three exonucleases is a 5' to 3' exonuclease and at least two of the at least three exonucleases are 3' to 5' exonucleases. The exonuclease mixture may comprise at least four exonucleases, wherein at least one of the at least four exonucleases is a 5' to 3' exonuclease and at least one of the at least four exonucleases is a 3' to 5' exonuclease. The exonuclease mixture may comprise at least four exonucleases, wherein at least two of the at least four exonucleases are 5' to 3' exonucleases and at least one of the at least four exonucleases is a 3' to 5' exonuclease. The exonuclease mixture may comprise at least four exonucleases, wherein at least one of the at least four exonucleases is a 5' to 3' exonuclease and at least two of the at least four exonucleases are 3' to 5' exonucleases. Preferably, the exonuclease mixture comprises at least four exonucleases, wherein at least two of the at least four exonucleases are 5' to 3' exonucleases and at least two of the at least four exonucleases are 3' to 5' exonucleases.

The 5' to 3' exonuclease(s) may be Lambda exonuclease and/or T7 exonuclease. The 3' to 5' exonuclease(s) may be exonuclease I and/or exonuclease III. The 5' to 3' exonuclease(s) may be exonuclease VIII.

The exonuclease mixture may comprise at least two exonucleases selected from a group of exonuclease I, exonuclease III, T7 exonuclease, and Lambda exonuclease. The exonuclease mixture may comprise at least three exonucleases selected from a group of exonuclease I, exonuclease III, T7 exonuclease, and Lambda exonuclease. Preferably, the exonuclease mixture comprises exonuclease I, exonuclease III, T7 exonuclease, and Lambda exonuclease.

The exonuclease mixture may comprise one exonuclease selected from Lambda exonuclease and/or T7 exonuclease, and one exonuclease selected from exonuclease I and/or exonuclease III. For example, the exonuclease mixture may comprise Lambda exonuclease and exonuclease I. For example, the exonuclease mixture may comprise Lambda exonuclease and exonuclease III. For example, the exonuclease mixture may comprise T7 exonuclease and exonuclease I. For example, the exonuclease mixture may comprise T7 exonuclease and exonuclease III.

The exonuclease mixture may comprise Lambda exonuclease, T7 exonuclease and exonuclease I. The exonuclease mixture may comprise Lambda exonuclease, T7 exonuclease and exonuclease III. The exonuclease mixture may comprise Lambda exonuclease, exonuclease I and exonuclease III. The exonuclease mixture may comprise T7 exonuclease, exonuclease I and exonuclease III.

The exonuclease mixture may further comprise exonucleases which are able to cleave DNA in both 3' to 5' and 5' to 3' directions. For example, the exonuclease mixture may comprise exonuclease V.

The ligase is preferably T7 DNA ligase.

The kit may additionally comprise a DNA polymerase, at least one buffer and/or a nuclease.

The kit may comprise at least:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1 and the second strand of the first and/or second adaptor molecule comprises a excisable nucleotide;
(b) an endonuclease;
(c) a ligase;
(d) a DNA glycosylase; and
(e) an exonuclease mixture.

The kit may comprise at least:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1 and the second strand of the first and/or second adaptor molecule comprises an abasic site;
(b) an endonuclease;
(c) a ligase;
(d) optionally an AP endonuclease; and
(e) an exonuclease mixture.

The kit may comprise at least:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1 and the second strand of the first and/or second adaptor molecule comprises a target sequence for a nicking endonuclease;
(b) an endonuclease;
(c) a ligase;
(d) a nicking endonuclease; and
(e) an exonuclease mixture.

The first strand of the first adaptor molecule may comprise the sequence of SEQ ID NO: 7 or a portion thereof. The second strand of the first adaptor molecule may comprise the sequence of SEQ ID NO: 8 or 9 or a portion thereof. The first strand of the second adaptor molecule may comprise the sequence of SEQ ID NO: 10 or a portion thereof. The second strand of the second adaptor molecule may comprise the sequence of SEQ ID NO: 11 or 12 or a portion thereof. The first and second adapter molecules may comprise a different nucleic acid sequence. The first and/or second strands of the adaptor molecules may comprise any of the sequences shown in the figures and in the table below.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The excisable nucleotide may be 8-oxoguanadine, 2,6-diamino-4-hydroxy-5-formamidopyrimidine (FapyG or FapyA), 3-methyladenine, 7-methylguanosine, hypoxanthine, xanthine or thymidine, uracil, thymine glycol, 5-hydroxycytosine (5-hC), 5-hydroxyuracil (5-hU), 3-methylguanine (3-meG), 3,N4-ethenocytosine or an ethylated base. The second strand may comprise an abasic site, also known as an AP site, which is an apurinic or apyrimidinic site in the DNA that does not have a purine or a pyrimidine.

The DNA glycosylase may be OGG1, MPG, SMUG1, UNG1, MBD4, TDG, MYH1, NTHL1, NEIL1, NEIL2 or NEIL3.

The nicking endonuclease may be Nt.BspQI, Nt.CviPII, Nt.BstNBI, Nb.BsrDI, Nb.Btsl, Nt.Alwl, Nb.BbvCI, Nt BbvCI, Nb.Bsml, Nb.BssSI and/or Nt.BsmAI
The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3!,Sap!, Aarl, Acc361, AclWI, Acul, Ajul, Alol, Alw261, Alwl, Arsl, AsuHPI, Bael, Barl, Bbvl, Bccl, BceAI, Bcgl, BciVI, BcoDI, BfuAl, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6!, BstF51, BstMAI, BstV11, BstV2!, Bsul, BtgZI, BtsCI, Btsl-v2, BtsMutl, Bve!, Csel, CspCI, Eam1104!, Earl, Ecil, Eco31I, Eco57!, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269!, NmeAIII, PaqCl, PciSI, Pctl, Plel, Ppsl, Psrl, Schl, SfaNI, Taqll, TspDTI and/or TspGWI restriction enzyme.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

### SEQUENCES

**Table 1. Sequences used in the present application. * indicates a phosphorothioated linkage, N is any of A, C, G or T. Specifically, * indicates a phosphorothioated linkage between two nucleotides. For example, "G*G" means that the phosphorothioated linkage is between the two G nucleotides. /5phos/ denotes a 5'-phosphate.**

| **SEQ ID NO.** | **Sequence 5'-3'** | **Description** |
|---|---|---|
| 1 | G*G*C*C*A*ACAAATGTTAG | First adaptor first strand |
| 2 | NNNNCTAACATTTGTTGGCC | First adaptor second strand |
| 3 | NNNNCTAACATTTGTT*G*G*C*C* | Second adaptor first strand |
| 4 | GGCCAACAAATGTTAG | Second adaptor second strand |
| 5 | G*G*C*C*A*ACAAATGTTAGNNNNN | First adaptor first strand ligated to the target DNA |
| 6 | NNNNNCTAACATTTGTTGGCC | First adaptor second strand ligated to the target DNA |
| 14 | NNNNNCTAACATTTGTT*G*G*C*C* | Second adaptor first stranded ligated to the target DNA |
| 15 | GGCCAACAAATGTTAGNNNNN | Second adaptor second strand ligated to the target DNA |
| 7 | N*ACAAATGTTAG | Exemplary first adaptor first strand |
| 8 | AGGGCTAACATTTGTN | Exemplary first adaptor second strand |
| 9 | AGGGCTAACATTTGTN* | Exemplary first adaptor second strand (with N*) |
| 10 | CATGCTAACATTTGN* | Exemplary second adaptor first strand |
| 11 | NCAAATGTTAG | Exemplary second adaptor second strand |
| 12 | N*CAAATGTTAG | Exemplary second adaptor second strand (with N*) |
| 13 | ATAACTTCGTATAGCATACATTATACGAAGTTAT | LoxP sequence |
| 16 | C*G*C*G*C*AGTGACACCCC | First adaptor first strand |
| 17 | ATCTGGGGTGTCACTGCGCG | First adaptor second strand |
| 18 | GGGAGGGTCGGGAC*A*A*A*G* | Second adaptor first strand |
| 19 | CTTTGTCCCGACCC | Second adaptor second strand |
| 20 | G*G*C*C*A*AGAAAaGTTAG | Uracil (first) adaptor first strand |
| 21 | /5phos/AGGGCTAAUUUACT*T*G*G*C*C | Uracil (first) adaptor second strand |
| 22 | G*G*C*C*A*AGTAAaGTTAG | unprotected adaptor first strand |
| 23 | /5phos/AGGGCTAACTTTACTTGGCC | unprotected adaptor second strand |
| 24 | G*G*C*C*A*AGTAAAGTTAG | SMUG specific adaptor 1 first strand |
| 25 | /5phos/AGGGCTATUGTTTUGTGGCC | SMUG specific adaptor 1 second strand |
| 26 | G*G*C*C*A*AATAAAATTAG | SMUG specific adaptor 2 first strand |
| 27 | /5phos/AGGGCTAAUTTTAUTTGGCC | SMUG specific adaptor 2 second strand |
| 28 | /5phos/TTTTCTAACATTTGT*T*G*G*C*C | Uracil second adaptor first strand |
| 29 | /5phos/G*G*C*C*A*ACAAATGTTAG | Uracil second adaptor second strand |
| 30 | /5phos/TTTTCTAACATTTGT*T*G*G*C*C | Unprotected (second) adaptor first strand |
| 31 | /5phos/GGCCAACAAATGTTAG | Unprotected (second) adaptor second strand |

### BRIEF DESCRIPTION OF THE DRAWINGS

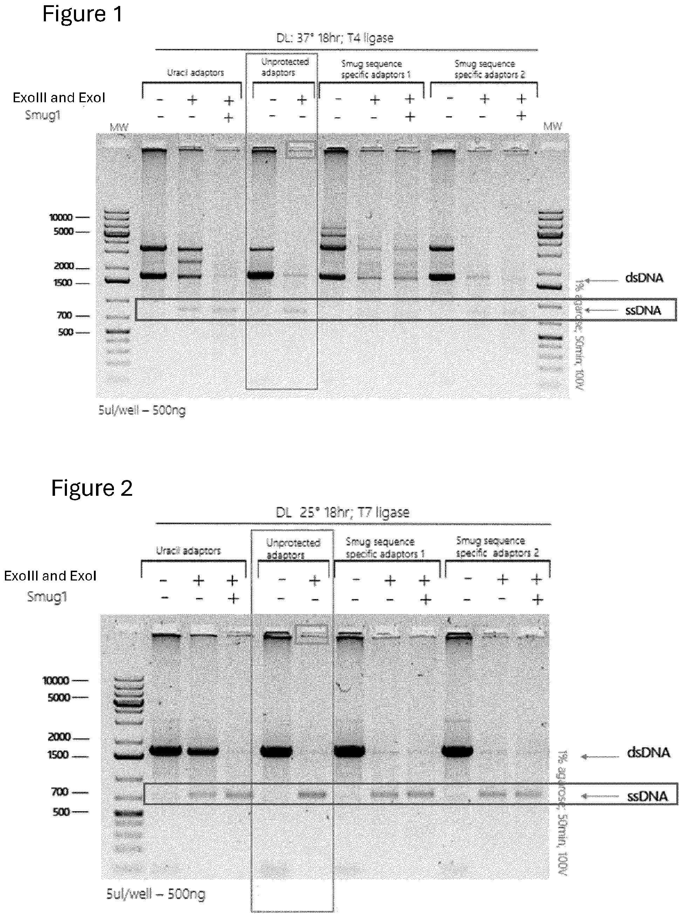

Figure 1 shows the results for ligation with T4 DNA ligase. The figure shows comparing the generation of ssDNA using T4 ligase and different adaptor types, in the presence of Smug1 and/or ExoIII/ExoI.
Figure 2 shows the results for ligation with T7 DNA ligase. The figure shows comparing the generation of ssDNA using T7 ligase and different adaptor types, in the presence of Smug1 and/or ExoIII/ExoI.

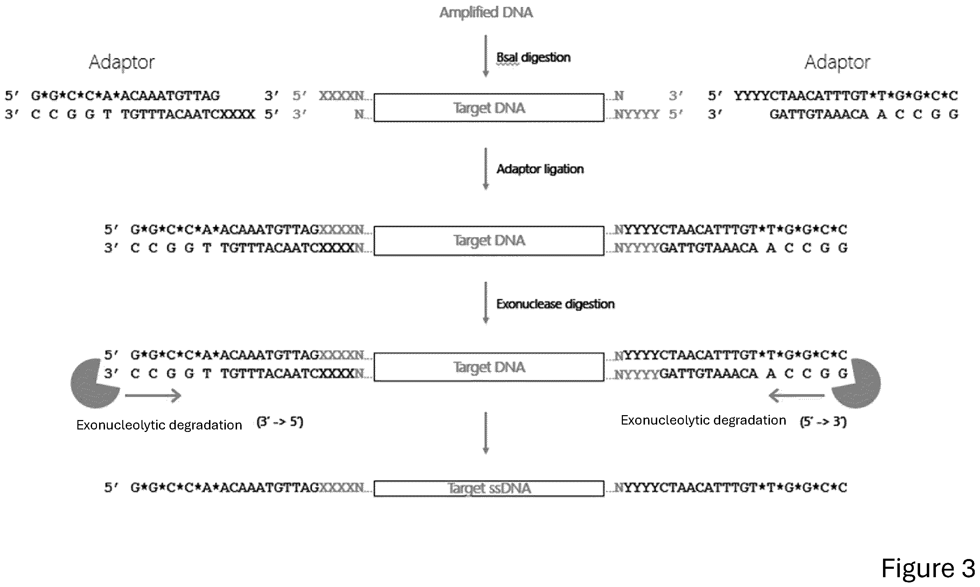

Figure 3 illustrates the sequences driving adaptor molecules ligation after Bsal digestion of an amplified double-stranded DNA molecule. Bsal digestion produces 4-nucleotide protruding ends at 5' at each side of the expression cassette. Upstream adaptors are formed by hybridization of complementary oligonucleotide containing phosphorothioate internucleotide linkages (marked as asterisks) in the 5' blunt end and forming a 4-nucleotide protruding end at 5'. Downstream adaptors are formed by hybridization of complementary oligonucleotides containing phosphorothioate internucleotide linkages (marked as asterisks) in the 3' blunt end and forming a 4-nucleotide protruding end at 5'. Complementary adaptor molecules are ligated at each side of the expression cassette, resulting in linear double stranded DNA product comprising protected nucleotides on both ends of one strand, while the complementary strand remains unprotected at both ends. Double-stranded exonucleases (e.g. E. coli exonuclease III, T7 exonuclease, T5 exonuclease, lambda exonuclease) can degrade the unprotected strand from both DNA ends (5' -> 3' and 3' -> 5'), resulting in single-stranded DNA comprising protected nucleotides on both ends.

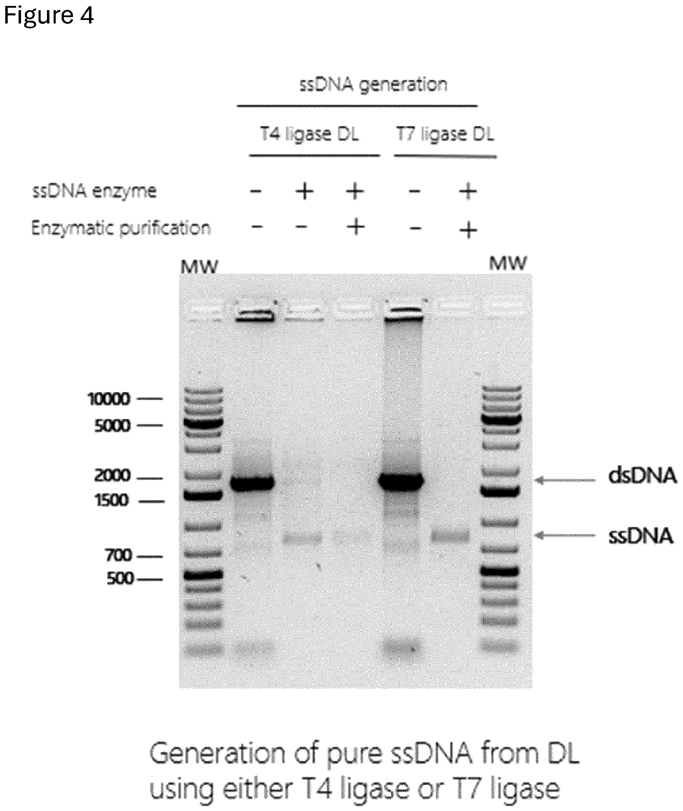

Figure 4 shows the yield and purity of the protected DNA using T7 DNA ligase and T4 DNA ligase.

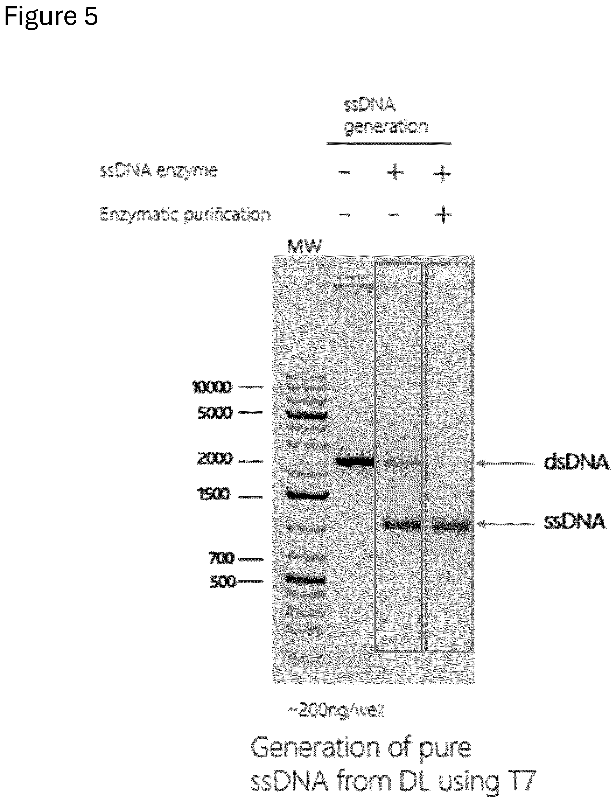

Figure 5 illustrates the purity of the final product after the combined treatment of (1) Exonuclease I and Exonuclease III (i.e. "ssDNA enzyme") (red box - lane 3), and (2) Exonuclease I, Exonuclease III, Lambda Exonuclease and T7 Exonuclease (i.e. both "ssDNA enzyme" and "enzymatic purification" enzymes) (green box - lane 4 (i.e. the last lane)).

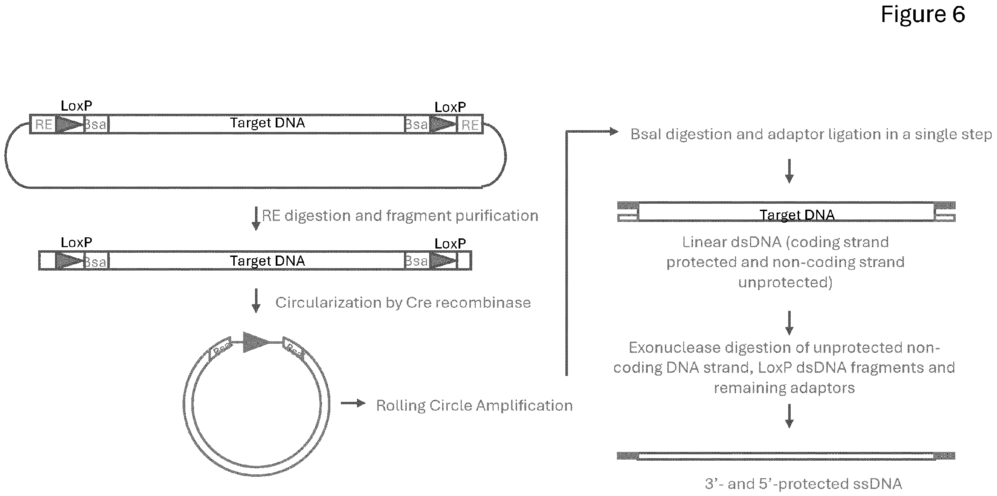

Figure 6 illustrates one example of the method of the invention. The figure illustrates the workflow to obtain 3' and 5' protected single-stranded DNA by digestion and ligation of adaptor molecules in a single step, starting from amplified double-stranded DNA molecule obtained through rolling circle amplification (RCA) of a circular DNA template generated through the action of Cre recombinase on substrates containing two LoxP sequences flanking the DNA of interest in the same direction.

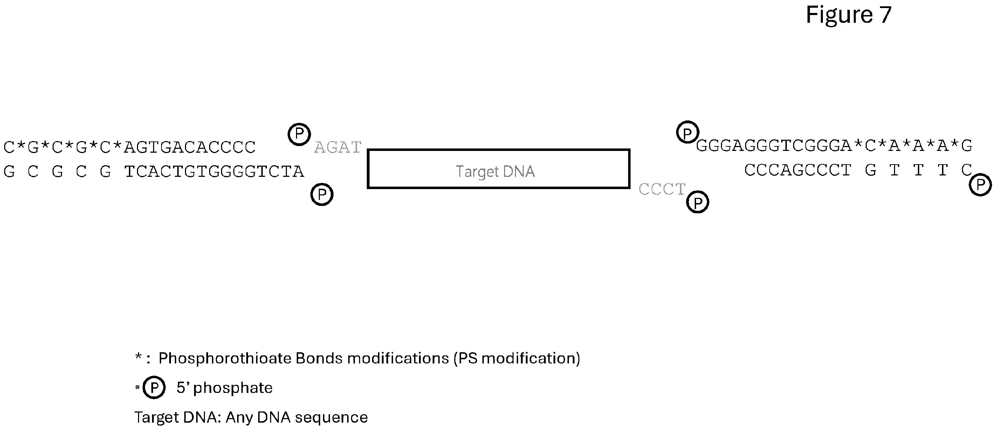

Figure 7 shows the exemplary position of phosphorothioation and phosphorylation in upstream and downstream adaptors used in one method for generating protected ssDNA. In the first strand (the top strand in the figure) the five 3' nucleotides and the five 5' nucleotides each have phosphorothioate linkages, shown by a *. The second strand (the bottom strand) has no modifications and is therefore susceptible to exonuclease digestion.

The invention is also defined in the following clauses:
1. A method for producing a protected DNA comprising a single-stranded DNA (ssDNA) cassette, wherein the method comprises:
   (a) generating a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1, wherein the step of generating the partially protected dsDNA comprises:
      (i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
      (ii) digesting the precursor dsDNA with the endonuclease to generate a digested precursor dsDNA;
      (iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1, and wherein the ligase is T7 DNA ligase; and
      (iv) ligating the first adaptor molecule to a first end of the digested precursor dsDNA and ligating the second adaptor molecule to a second end of the digested precursor dsDNA thereby generating the partially protected dsDNA; and
   (b) digesting the second strand of the partially protected dsDNA with an exonuclease mixture thereby generating the protected DNA, wherein the exonuclease mixture comprises at least a 5' to 3' exonuclease and a 3' to 5' exonuclease.
2. A method for producing a protected DNA comprising a single-stranded DNA (ssDNA) cassette, wherein the method comprises:
   (a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
   (b) digesting the second strand of the partially protected dsDNA with an exonuclease mixture thereby generating the protected DNA, wherein the exonuclease mixture comprises at least three exonucleases, wherein at least one of the at least three exonucleases is a 5' to 3' exonuclease and at least one of the at least three exonucleases is a 3' to 5' exonuclease.
3. The method of clause 1 or clause 2, wherein the exonuclease mixture comprises at least two 5' to 3' exonucleases.
4. The method of any one of clauses 1-3, wherein the exonuclease mixture comprises at least two 3' to 5' exonucleases.
5. The method of any one of clauses 1-4, wherein the exonuclease mixture comprises at least four exonucleases, optionally wherein at least two of the at least four exonucleases are 5' to 3' exonucleases and at least two of the at least four exonucleases are 3' to 5' exonucleases.
6. The method of any one of clauses 1-5, wherein the 5' to 3' exonuclease is Lambda exonuclease and/or T7 exonuclease.
7. The method of any one of clauses 1-6, wherein the 3' to 5' exonuclease is exonuclease I and/or exonuclease III.
8. The method of clause 5, wherein the at least four exonucleases are Lambda exonuclease, T7 exonuclease, exonuclease I, and exonuclease III.
9. The method of clause 2, wherein the method further comprises a step of generating the partially protected dsDNA.
10. The method of clause 9, wherein the step of generating the partially protected dsDNA comprises:
   (i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
   (ii) digesting the precursor dsDNA with the endonuclease to generate a digested precursor dsDNA;
   (iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1; and
   (iv) ligating the first adaptor molecule to a first end of the digested precursor dsDNA and ligating the second adaptor molecule to a second end of the digested precursor dsDNA thereby generating the partially protected dsDNA.
11. The method of clause 1 or clause 10, wherein steps (i)-(iv) are performed in a single contiguous aqueous volume.
12. The method of clause 11, wherein step (b) comprising contacting the single contiguous aqueous volume with the exonuclease mixture without purifying the partially protected dsDNA.
13. The method of clause 10, wherein the ligase is a T7 DNA ligase.
14. The method of any one of clauses 1 or 9-13, wherein the method further comprises:
   amplifying a DNA template to generate the precursor dsDNA, wherein the DNA template comprises the cassette and the endonuclease target sequences, optionally wherein the DNA template is amplified by rolling circle amplification.
15. The method of anyone of clauses 1-14, further comprising the step of nicking the second strand of the partially protected dsDNA prior or at the same time as digesting the second strand of the partially protected dsDNA, optionally wherein the nicking is performed by a DNA glycosylase, a nicking endonuclease or an AP endonuclease.

### EXAMPLES

The inventors of the present application improved the methods described in WO2024/017978 (which is incorporated herein by reference).

In summary, a precursor DNA is generated by rolling circle amplification (RCA), digested and then ligated with two adaptors (one in each side of the DNA) in a single-step, single-volume reaction.

A plasmid containing an expression cassette was subjected to the procedure described below.

Cre recombinase from the P1 bacteriophage is a Type I topoisomerase. The enzyme catalyzes the sitespecific recombination of DNA between loxP sites. LoxP recognition site (34 bp) consists of two 13 bp inverted repeats which flank an 8 bp spacer region, which confers directionality. The products of Cremediated recombination are dependent upon the location and relative orientation of the loxP sites. Two DNA species containing single loxP sites were fused. DNA found between two loxP sites oriented in the same direction was excised as a circular loop of DNA, while DNA between opposing loxP sites was inverted with respect to external sequences. Cre recombinase requires no additional cofactors or accessory proteins for its function.

**Cre reaction conditions:** reaction volume 1 ml, DNA of interest purified after restriction enzyme digestion (2 ng/µl), Cre recombinase (NEB, 0,08 units/µl), incubation time and temperature: 30 min at 37°C and 20 min at 80°C. Next, to remove remaining non-circular DNA molecules before the amplification step, *E. coli* exonuclease I (NEB, 0,4 units/µl) and III (NEB, 2 units/µl) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like TthPrimPol (Picher *et al,* 2016) that synthesizes the primers for Phi29DNApol during the amplification reaction.

**Rolling Circle Amplification (RCA):** before the amplification, circularized DNA was first denatured by adding 1 volume of buffer D (400 mM KOH, 10 mM EDTA) and incubating 3 min at room temperature. The sample was then neutralized by adding 1 volume of buffer N (400 mM HCl, 600 mM Tris-HCl pH 7.5). Rolling circle amplification conditions: 20 ml reaction volume, 2 ml TruePrime WGA reaction buffer 10x (4basebio), 3 ml denatured DNA sample, 2 ml TthPrimPol (1 µM), 320 µl QualiPhi Phi29DNApol (12,5 µM), 5 units PPase (Thermo) and 2 ml dNTPs (10 mM). Incubation time and temperature: 20 hours at 30°C and 10 min at 65°C.

**DNA digestion and adaptor ligation:** amplified DNA was then incubated with Type II restriction enzyme Bsal, T4 DNA ligase and complementary adaptors as defined herein to the 5' protruding ends generated by Bsal on the amplified DNA. Digestion and ligation reaction conditions: reaction volume 20 ml, 2 ml reaction buffer, T4 DNA ligase 10x (NEB), 240 ng/µl amplified DNA, 0,6 units/µl Bsal-HFv2 (NEB), 20 units/µl T4 DNA ligase (NEB), DNA adaptors (1:10 molar excess), incubation time and temperature: 23 hours at 30°C.

As shown in Figure 7, the adaptor may comprise:
- a 5' single-stranded overhang (alternatively, the adaptor may comprise a 3' single-stranded overhang (not shown in this example));
- a phosphorylation modification at 5' of the overhang end of the adaptor; and
- a blunt end comprising exonuclease-resistant nucleotides in one of the strands.

The single-stranded overhang of the adaptor is complementary to the gene of interest (GOI).

The adaptors are supplied at molar excess, ranging from 1:2 to 1:20 molar ratio, in comparison to the molarity of RCA tandem repeats.

The concentration of DNA generated by RCA is measured (Qubit) in its concatemer form. The expected molarity of unique, tandem repeats is determined based on the length of a single copy of DNA sequence and the DNA concentration.

The digestion/ligation reaction may be performed in a single reaction volume containing:
a) digestion/ligation reaction buffer (50 mM Tris HCl [pH 7.5], 10 mM MgCl2, 10 mM DTT),
b) RCA material (240ng DNA/uL, n uM), 10mM ATP,
c) upstream adaptors (usually 2-5x n uM), and downstream adaptors (usually 5-20x n uM), The upstream and downstream adaptors are typically the same.
d) T4 or T7 Ligase (e.g. 33.2 ng/uL (construct dependent), 4basebio), and
e) Bsal or other endonuclease with a restriction site represented in the RCA concatemer (3.2 ng/uL Bsal).

The reaction is incubated at 37°C or 25°C for 20 hours and treated with an exonuclease mixture (comprising exonuclease I, III, and optionally T7 exonuclease and Lambda exonuclease) in order to remove any non-ligated DNA material or unprotected strand to generate the single-stranded DNA (ssDNA).

To generate the ssDNA (under the improved conditions - see examples 2 and 3), the reaction is performed in a single reaction volume containing:
Cutsmart Buffer from NEB (50 mM Potassium Acetate, 20 mM Tris-acetate, 10 mM Magnesium Acetate, 100 µg/ml BSA)
DNA material (240ng DNA/uL, n nM, before any exonuclease treatment) ("n" is different based on the size of the construct, two constructs were used here; 1) 2.2 kb, where "n" was 168nM and 2) 1.6kb, where "n" was 230nM)
Exonuclease I (4basebio)
Exonuclease III (4basebio)
T7 exonuclease (NEB)
Lambda Exonuclease (NEB)

The reaction is incubated at 37°C in order to remove any non-ligated DNA material with the exonucleases listed above.

### Example 1 - Ligase comparison

Protected DNA was produced as described above. Here, the use of two different DNA ligases was compared - T4 DNA ligase and T7 DNA ligase.

### Figure 1

A DNA construct of -1600bp was amplified using rolling circle amplification (RCA) with QualiPhi Phi29 DNA polymerase (4basebio). The reaction was incubated at 30°C for 20 hours. The RCA reaction product was then digested and ligated to adaptors in a single reaction using Bsal and T4 ligase.

The first strategy to generate the ssDNA protected product involved the use of two different enzymes: an Uracil N-glycosylase (SMUG1), which removes the uracil base by cleaving the covalent bond with deoxyribose, without breaking the phosphodiester backbone, and Exonuclease III (Exolll) (which can digest dsDNA specifically; Exolll has apurinic/apyrimidinic (AP)-endonuclease activity in addition to the 3'->5' exonuclease activity. The AP endonuclease activity results in AP site incision, which generates a nick). In this strategy, the uracil adaptors used during digestion/ligation included phosphorothioate (PS) modifications in both strands of each adaptor, and a single or multiple uracil bases in one of the strands (e.g. bottom) of one of the adaptors. The dsDNA product therefore included PS modifications in both strands. For the Smug sequence specific adaptors used during digestion/ligation included phosphorothioate (PS) modifications in the top strand (only) and multiple uracil bases in the bottom strand of one of the adaptors. The dsDNA product therefore included

PS modifications in the top strand (only). The action of SMUG1 and Exolll/Exol allowed then for the generation of a ssDNA protected product.

The second strategy to generate the ssDNA protected product involved using only Exolll and Exol to generate the protected ssDNA product. In this approach, the adaptors used during digestion/ligation comprised PS modification in the top strand (only). This allowed the generation of a final dsDNA molecule which has one strand protected and the other one unprotected. The action of Exolll and Exol digested both the 3' and 5' ends of the unprotected strand generating a ssDNA protected product.

### Results:

As can be seen in Figure 1, T4 ligase is used to generate final ssDNA molecule. 4 different adaptors (Uracil adaptor, Unprotected adaptor, SMUG1 sequence specific adaptor 1 and SMUG1 sequence specific adaptor 2) were used. Digestion took place using Exol/III and/or Smug 1 enzymes as indicated in the figure. The gel results confirm that ssDNA molecules can be generated using both strategies described above. However, the design of the adaptors seems to have an impact on the purity of the final product (Blue box - i.e. long horizontal box). The red box (i.e. the long vertical box) demonstrates the efficiency of the second strategy to generate a ssDNA molecule without the need for an additional enzyme such as SMUG1. However, with both strategies residual dsDNA is observed.

### Uracil Adaptor (left):

| | |
|---|---|
| 5' G*G*C*C*A*ACAAATGTTAG 3' | (SEQ ID NO: 20) |
| 3' C*C*G*G*T*TG**UUU**ACAATCGGGA /5phos/ 5' | (SEQ ID NO: 21) |

### Unprotected adaptor (left):

5' G*G*C*C*A*AGTAAaGTTAG 3' (SEQ ID NO: 22)
3' CCGGTTCATTTCAATCGGGA /5phos/ 5' (SEQ ID NO: 23)

### SMUG specific Adaptor 1:

| | |
|---|---|
| 5' GGCCACGAAACGATAG 3' | (SEQ ID NO: 24) |
| 3' CCGGT**GUT**TT**GUT**ATCGGGA /5phos/ 5' | (SEQ ID NO: 25) |

### SMUG specific Adaptor 2:

| | |
|---|---|
| 5' GGCCAAGTAAAGTTAG 3' | (SEQ ID NO: 26) |
| 3' CCGGT**TUA**TT**TUA**ATCGGGA /5phos/ 5' | (SEQ ID NO: 27) |

### Uracil Adaptors (right) (applies also to SMUG specific Adaptor 1 and 2):

3' C*C*G*G*T*TGTTTACAATCTTTT/5phos/ 5' (SEQ ID NO: 28)
5' /5phos/G*G*C*C*A*ACAAATGTTAG 3' (SEQ ID NO: 29)

### Unprotected adaptor (right):

3' C*C*G*G*T*TGTTTACAATCTTTT/5phos/ 5' (SEQ ID NO: 30)
5' 5phos/GGCCAACAAATGTTAG 3' (SEQ ID NO: 31)

### Figure 2

The DNA construct of -1600bp was amplified using rolling circle amplification (RCA) with QualiPhi Phi29 DNA polymerase (4basebio). The reaction was incubated at 30°C for 20 hours. The RCA reaction product was then digested and ligated to the adaptors in a single reaction using Bsal and T7 ligase (as in Example sections above).

Protected ssDNA product was generated using two different strategies as discussed for Figure 1 above.

### Results:

As can be seen in Figure 2, T7 ligase was used in all reactions to generate the final ssDNA molecule. 4 different adaptors (Uracil adaptor, Unprotected adaptor, SMUG1 sequence specific adaptor 1 and SMUG1 sequence specific adaptor 2) were used. The gel results confirm that the protected ssDNA product can be generated using both strategies described above. However, the design of the adaptors seems to have an impact on the purity of the final product (Blue box - i.e. long horizontal box). The red box (i.e. the vertical box) demonstrates the efficiency of the second strategy to generate a ssDNA molecule without the need for a second enzyme, such as SMUG1. However, with both strategies residual dsDNA is observed. It is important to note that the use of T7 ligase significantly improves the yield as compared to the use of T4 ligase.

Figures 1 and 2 show the results for T4 DNA ligase and T7 DNA ligase, respectively. In the Figures, the blue box (i.e. the long horizontal box) highlights the ssDNA protected product. As shown in Figures 1 and 2, the yield (intensity) of the ssDNA band is higher when using T7 ligase compared to T4 ligase.

Moreover, the intensity of the residual dsDNA was lower when T7 ligase was used.

Therefore, the T7 ligase yield was higher than the T4 ligase. Similarly, the ratio ssDNA/dsDNA was higher when T7 ligase was used compared to T4 ligase.

### Example 2 - Improvement of purity

The DNA construct of -1600bp was amplified using rolling circle amplification (RCA) with QualiPhi Phi29 DNA polymerase (4basebio). The reaction was incubated at 30°C for 20 hours. The RCA reaction product was then digested and ligated in single reaction using Bsal and T4 or T7 ligase and "unprotected" adaptors (left and right) (see Example 1 - SEQ ID Nos: 22, 23 and 30, 31). When correctly ligated, the final dsDNA molecule will comprise phosphorothioate modifications on both ends of one (e.g. top) strand, which confers protection against exonucleases, and one (e.g. bottom) unprotected strand, which will be digested by exonucleases.

Figure 3 describes the general approach in the present example. Figure 3 illustrates the sequences driving adaptor molecules ligation after Bsal digestion of an amplified double-stranded DNA molecule. Bsal digestion produces 4-nucleotide protruding ends at each side of the expression cassette. Upstream adaptors are formed by hybridization of complementary oligonucleotides (e.g. SEQ ID NO: 1, 2) containing phosphorothioate internucleotide linkages (marked as asterisks) in the 5' end of the top strand and forming a 4-nucleotide protruding end at 5' of the bottom strand. Downstream adaptors are formed by hybridization of complementary oligonucleotides (e.g. SEQ ID NO: 3, 4) containing phosphorothioate internucleotide linkages (marked as asterisks) in the 3' end of the top strand and forming a 4-nucleotide protruding end at 5' of the top strand. The 5' blunt end, for example, is on the top or bottom strand. Complementary adaptor molecules are ligated at each side of the expression cassette, resulting in a linear double stranded DNA product comprising protected nucleotides on both ends of one strand, while the complementary strand remains unprotected on both ends. Double-stranded exonucleases (e.g. E. coli exonuclease III, T7 exonuclease, T5 exonuclease, lambda exonuclease) can degrade the unprotected strand from both DNA ends (5' -> 3' and 3' -> 5'), resulting in a single-stranded DNA product comprising protected nucleotides on both ends.

As in Example 2, Exolll and Exol were used to generate the ssDNA protected product (denoted "ssDNA enzyme" in Figure 4). The additional exonucleases, T7 exonuclease and Lambda exonuclease (denoted "enzymatic purification" in Figure 4), were also tested to see if they improved the purity of the final product.

Figure 4 shows that the T7 ligase yield was higher than T4 ligase and also the final product (ssDNA protected product) after the exonuclease mixture treatment has better yield when T7 DNA ligase was used. Moreover, the overall quality and purity of the ssDNA protected product was much higher when T7 ligase was used compared to the T4 ligase.

To confirm that the T7 ligase in combination with Exonuclease I, Exonuclease III, Lambda Exonuclease and T7 Exonuclease mixture performs better than T4 ligase with the same exonuclease mixture, these two experimental conditions were tested side by side.

### Example 3- Testing larger construct

A DNA construct of ~2200bp was amplified using rolling circle amplification (RCA) with QualiPhi Phi29 DNA polymerase (4basebio). The reaction was incubated at 30°C for 20 hours. The RCA reaction product was then digested and ligated in single reaction using BsmBI and T7 ligase and "unprotected" adaptors (left and right) (see Example 1 - SEQ ID Nos: 22, 23 and 30, 31). When correctly ligated, the final dsDNA molecule will comprise phosphorothioated modifications on both ends of one (e.g. top) strand, which confers protection against exonucleases, and one (e.g. bottom) unprotected strand which will be digested by exonucleases.

Different exonucleases, including Exonuclease I, Exonuclease III, Lambda Exonuclease and T7 Exonuclease were tested with the aim to remove any smear or products present in the well (see the green box (i.e. the small horizontal box in lane 6) in Figures 1 and 2).

Using each exonuclease individually did not yield an improvement in purity (not shown). However, when Exonuclease I and Exonuclease III were used together an improvement was observed to purity (lane 3 in Figure 5). The best improvement to the purity of the final product was observed upon the combined use of Exonuclease I, Exonuclease III, Lambda Exonuclease and T7 Exonuclease (lane 4 in Figure 5).

Figure 5 illustrates the purity of the final product after the combined treatment of (1) Exonuclease I and Exonuclease III (i.e. "ssDNA enzyme") (red box; i.e. lane 3), and (2) Exonuclease I, Exonuclease III, Lambda Exonuclease and T7 Exonuclease (green box; i.e. lane 4 (the last lane)). In Figure 5, Exolll and Exol are denoted "ssDNA enzyme" and the additional exonucleases (T7 exonuclease and Lambda exonuclease) are denoted "enzymatic purification". As can be seen in the red box, where only Exonuclease I and Exonuclease III were used, a proportion of the dsDNA and some extra bands (smear) persists; however, after using Exonuclease I, Exonuclease III, Lambda Exonuclease and T7 Exonuclease combination (green box), the dsDNA along with the unwanted bands are fully digested and the purity of the ssDNA product has been improved significantly.

The results confirm the efficacy of T7 ligase to generate the final ssDNA molecule with a larger size construct. The results also confirm the impact of the extra treatment with T7 exonuclease and Lambda exonuclease on the purity of the final protected ssDNA product. The gel results confirm that protected ssDNA products can be generated with high yield using T7 ligase. It is also confirmed that the extra treatment with T7 exonuclease and Lamda exonuclease improves significantly the purity and removes any residual dsDNA which is still present when only Exolll and Exol are used.

## Claims

1. A method for producing a protected DNA comprising a single-stranded DNA (ssDNA) cassette, wherein the method comprises:
(a) generating a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1, wherein the step of generating the partially protected dsDNA comprises:
(i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(ii) digesting the precursor dsDNA with the endonuclease to generate a digested precursor dsDNA;
(iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1, and wherein the ligase is T7 DNA ligase; and
(iv) ligating the first adaptor molecule to a first end of the digested precursor dsDNA and ligating the second adaptor molecule to a second end of the digested precursor dsDNA thereby generating the partially protected dsDNA; and
(b) digesting the second strand of the partially protected dsDNA with an exonuclease mixture thereby generating the protected DNA, wherein the exonuclease mixture comprises at least a 5' to 3' exonuclease and a 3' to 5' exonuclease.

2. A method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease mixture thereby generating the protected DNA, wherein the exonuclease mixture comprises at least three exonucleases, wherein at least one of the at least three exonucleases is a 5' to 3' exonuclease and at least one of the at least three exonucleases is a 3' to 5' exonuclease.

3. The method of claim 1 or claim 2, wherein the exonuclease mixture comprises at least two 5' to 3' exonucleases.

4. The method of any one of claims 1-3, wherein the exonuclease mixture comprises at least two 3' to 5' exonucleases.

5. The method of any one of claims 1-4, wherein the exonuclease mixture comprises at least four exonucleases, optionally wherein at least two of the at least four exonucleases are 5' to 3' exonucleases and at least two of the at least four exonucleases are 3' to 5' exonucleases.

6. The method of any one of claims 1-5, wherein the 5' to 3' exonuclease is Lambda exonuclease and/or T7 exonuclease.

7. The method of any one of claims 1-6, wherein the 3' to 5' exonuclease is exonuclease I and/or exonuclease III.

8. The method of claim 5, wherein the at least four exonucleases are Lambda exonuclease, T7 exonuclease, exonuclease I, and exonuclease III.

9. The method of claim 2, wherein the method further comprises a step of generating the partially protected dsDNA.

10. The method of claim 9, wherein the step of generating the partially protected dsDNA comprises:
(i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(ii) digesting the precursor dsDNA with the endonuclease to generate a digested precursor dsDNA;
(iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1; and
(iv) ligating the first adaptor molecule to a first end of the digested precursor dsDNA and ligating the second adaptor molecule to a second end of the digested precursor dsDNA thereby generating the partially protected dsDNA.

11. The method of claim 1 or claim 10, wherein steps (i)-(iv) are performed in a single contiguous aqueous volume.

12. The method of claim 11, wherein step (b) comprising contacting the single contiguous aqueous volume with the exonuclease mixture without purifying the partially protected dsDNA.

13. The method of claim 10, wherein the ligase is a T7 DNA ligase.

14. The method of any one of claims 1 or 9-13, wherein the method further comprises:
amplifying a DNA template to generate the precursor dsDNA, wherein the DNA template comprises the cassette and the endonuclease target sequences, optionally wherein the DNA template is amplified by rolling circle amplification.

15. The method of anyone of claims 1-14, further comprising the step of nicking the second strand of the partially protected dsDNA prior or at the same time as digesting the second strand of the partially protected dsDNA, optionally wherein the nicking is performed by a DNA glycosylase, a nicking endonuclease or an AP endonuclease.
